(19) Europäisches Patentamt
European Patent Office
Office européen des brevets

(11) **EP 3 149 148 B1**

(12) **EUROPÄISCHE PATENTSCHRIFT**

(45) Veröffentlichungstag und Bekanntmachung des Hinweises auf die Patenterteilung:
**01.08.2018 Patentblatt 2018/31**

(21) Anmeldenummer: **15726908.5**

(22) Anmeldetag: **26.05.2015**

(51) Int Cl.:
*C12M 1/107* (2006.01)     *C12M 3/00* (2006.01)
*C12M 1/00* (2006.01)     *C12M 1/40* (2006.01)
*C12M 1/34* (2006.01)

(86) Internationale Anmeldenummer:
**PCT/EP2015/061605**

(87) Internationale Veröffentlichungsnummer:
**WO 2015/181180 (03.12.2015 Gazette 2015/48)**

(54) **PRODUKTIONSVERFAHREN UND VORRICHTUNG ZUR MIKROBIOLOGISCHEN UMSETZUNG VON GASEN**

PRODUCTION METHOD AND APPARATUS FOR MICROBIOLOGICALLY CONVERTING GASES

PROCÉDÉ DE PRODUCTION ET DISPOSITIF DE CONVERSION MICROBIOLOGIQUE DE GAZ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.05.2014 DE 102014107377**

(43) Veröffentlichungstag der Anmeldung:
**05.04.2017 Patentblatt 2017/14**

(73) Patentinhaber: **MWK Bionik GmbH**
**83093 Bad Endorf (DE)**

(72) Erfinder:
• **UPHOFF, Christian**
**83229 Aschau im Chiemgau (DE)**
• **VOLL, Judith**
**83229 Aschau (DE)**

(74) Vertreter: **Stolmár & Partner**
**Patentanwälte PartG mbB**
**Blumenstraße 17**
**80331 München (DE)**

(56) Entgegenhaltungen:
WO-A1-2008/154301     WO-A1-2009/118445
WO-A2-2009/010959     US-A1- 2011 244 538
US-B1- 6 492 135

EP 3 149 148 B1

**Beschreibung**

Gebiet der Erfindung

Die Erfindung betrifft ein mikrobiologisches Produktionsverfahren und Vorrichtungen zur Umsetzung von Gasen zu biotechnologischen Produkten.

Stand der Technik

Ressourcenknappheit und Nachhaltigkeitsbedarf verlangen nach Verfahren, die es erlauben, neben fossilen Rohstoffen wie Rohöl und Erdgas auch Reststoffe als alternative Rohstoffquellen für organische Kohlenstoffverbindungen zu nutzen. Als Kohlenstoffquellen, die keine Konkurrenz zu Nahrungsmitteln darstellen stehen kohlenstoffhaltige Gase zur Verfügung, die entweder gezielt aus organischen Reststoffen gewonnen werden, als Reststoffe in der Industrie entstehen oder auch natürlichen Ursprungs sind. Als Beispiele für solche Gase sind zu nennen Synthesegas, ein Gasgemisch variierender Anteile von Kohlenmonoxid, Wasserstoff und Kohlendioxid, das entweder durch Vergasungsmethoden direkt aus organischen Reststoffen erzeugt wird oder als industrielles Abfallprodukt anfällt, einzelne Kohlenoxide, die in der Industrie oder bei Endverbrauchern durch oxidative Prozesse an organischen Kohlenstoffverbindungen als Abfallstoffe erzeugt werden, oder auch Erdgasvorkommen, industriellen oder natürlichen Ursprungs, die freigesetzt in die Atmosphäre, durch die vergleichsweise hohen Treibgaseffekte enorme Schäden verursachen.

Neben herkömmlichen chemischen Produktionsverfahren gewannen in den letzten Jahren biokatalytische Produktionsverfahren zur Verwertung gasförmiger Edukte zunehmend an Bedeutung. Die Nutzung natürlich optimierter Eigenschaften bietet große Vorteile gegenüber chemischen Standardverfahren. So weisen Biokatalysatoren vergleichsweise hohe Resistenz gegenüber prozessbegleitenden Störstoffen auf und zeichnen sich durch hohe Prozessstabilität aus. Ihre Funktionsbereiche weisen vergleichsweise niedrige Temperatur- und Druckbereiche auf, wodurch der Energiebedarf im Vergleich zu herkömmlichen chemischen Verfahren deutlich herabgesetzt wird. Hohe Selektivität und Spezifität, sowie hohe Produktausbeuten sind weitere Vorteile von Biokatalysatoren. Aufgrund dieser hervorragenden Eigenschaften werden biokatalytische Verfahren als wirtschaftlich rentable Alternativen diskutiert, die es in Zukunft erlauben werden, auch Gasvorkommen geringerer Mengen als Eduktquellen für die Produktion organischer Kohlenstoffverbindungen zu nutzen.

Kommerzielle Anwendung der Produktion kohlenstoffhaltiger Produkte aus gasförmigen Substraten findet derzeit auf unterschiedlichen Gebieten statt. INEOS Bio, Coskata und Lanzatech haben Fermentationsverfahren entwickelt, die sich derzeit im Stadium der Markteinführung befinden, die aus Synthesegas Alkohole und Ester produzieren. Derzeit werden diese Verfahren weiterentwickelt, um auch andere Produkte zu erzeugen. Calysta Energy LLC hat biotechnologische Verfahren entwickelt, mit denen aus Methan Methanol produziert wird. Weitere Firmen haben Verfahren für die biophotokatalytische Umsetzung von $CO_2$ zu Biomasse oder auch chemischen Produkten wie Alkohol entwickelt.

Verschiedene Produktionsverfahren für mikrobiologische Umsetzungen von Gasen zu gewünschten Produkten existieren und werden eingesetzt. Diese Verfahren können in zwei Verfahrensansätze unterteilt werden, die sich in dem vorliegenden Milieu der aktiven Mikrobiologie unterscheiden.

In einem ersten Verfahrensansatz schwebt die Mikrobiologie frei in der flüssigen Phase. Zumeist werden als Biokatalysatoren lebendige mikroorganische Zellen wie Bakterien, Hefen, Pilze oder Algen verwendet, die dazu fähig sind die Eduktgase im Rahmen ihres Stoffwechselkreislaufes als Nahrungsquelle zu verwenden. Die Begriffe Gas, Eduktgas und reaktives Eduktgas werden für die Zwecke der vorliegenden Beschreibung austauschbar füreinander Benutzt, falls nicht explizit anders vermerkt. Die umgebende meist wässrige Lösung enthält essentielle Spurenelemente, Makronährstoffe, Vitamine und andere Substanzen, die den Stoffwechselkreislauf unterstützen, oder geeignete Prozessparameter wie pH-Wert, Redoxpotential und Salinität sicherstellen. Die Eduktgase werden dem Prozess durch Einleiten in die Lösung zugeführt. Die fermentative Umsetzung der Edukte findet nach Einleiten der Gase in die Flüssigkeit in der flüssigen Phase statt.

Dieser Verfahrensansatz findet in unterschiedlichen Reaktoren Anwendung. Beispiele hierfür sind der kontinuierlich gerührte Reaktor (CSTR - *continuous stirred tank reactor, static mixer*) und andere, wie der *microbubble dispersion stirred tank reactor* oder der *bubble column reactor, gas lift reactor*. Je nach Reaktortyp werden unterschiedliche Verfahren genutzt, um den erforderlichen Massentransfer der Eduktgase zu den Biokatalysatoren zu begünstigen. Im CSTR sind es Rührvorrichtungen, die eine gleichmäßige Durchmischung der Lösung mit Gasblasen sicherstellen und gegebenenfalls die Gas-Wasser-Grenzfläche durch Platzenlassen der Gasblasen weiter vergrößern. Weitere genutzten Mechanismen sind die Einbringung der Gase durch Vorrichtungen, die fein zerteilte Mikrobläschen erzeugen (microbubble dispersion stirred tank reactor), oder auch durch die Gestalt der Reaktoren eine möglichst energiearme Durchmischung von Gasblasen und Flüssigkeit mit langen Lebenszeiten der Gasblasen ermöglichen (bubble column reactor, gas lift reactor).

Beispielsweise wird in der Patentanmeldung US 2015/0031099 A1 ein Reaktor beschrieben, in dem das umzusetzende Gas in einen mit einer Fermentationsbrühe gefüllten Reaktor direkt in die Fermentationsbrühe eingeführt und mit dieser mittels Pumpe durch ein aufsteigendes und damit verbundenes absteigendes Rohr durchgeleitet wird. Dabei entstehende größere Gasblasen werden durch Fermentationsbrühe in kleinere zerplatzen gelassen und die dem Kreislauf entzogen wird und als mehrere Flüssigkeitsströme mittels einer Dusche auf den Schaum abgegeben wird, der auf der Flüssigkeitsoberfläche im aufsteigendem Rohr liegt. Der Gastransfer in die Fermentationsbrühe und zu den Mikroorganismen wird gemäß der Anmeldung durch den Transport von beiden im beschriebenen Kreislauf und durch Verkleinerung der Gasblasen bei deren Zerplatzen auf dem Schaum erreicht.

Diese Verfahrensform weist Vorteile in ihrer einfachen Handhabung auf und durch den geringen verfahrenstechnische Aufwand. Die hierdurch erzielte Prozessstabilität ist einer der wohl wichtigsten Vorteile dieser Verfahren. So kann der Prozess einfach anhand installierter Messvorrichtungen beobachtet werden. Der Ausgleich von gleichgewichtsstabilisierenden Prozessparametern durch Zugabe von Additiven erfolgt leicht. Der erforderliche Flüssigkeitsaustausch zur Abtrennung der erzeugten Produkte ist entweder kontinuierlich oder in Chargen möglich. Ebenfalls kann abgestorbene Biomasse leicht aus dem Prozess entfernt werden und so eine Kontamination durch entstehende Foulingprozesse vermieden werden.

Allerdings ist meist die Reaktivität eingeschränkt aufgrund geringer Massetransferkoeffizienten und niedriger Populationsdichten. Durch die verhältnismäßig geringen Ausmaße der Gas-Flüssigkeitsgrenzflächen, die weiter durch hohe Diffusionswiderstände in angrenzenden Zonen gekennzeichnet sind ergeben sich geringe Konzentrationen gelöster Gase. Zwar kann durch gute Durchmischung der Lösung der ständige Ausgleich der Konzentrationen bei Verbrauch von Eduktgasen sichergestellt werden, doch findet dieser Ausgleich auf relativ niedrigem Niveau statt. Der hohe Energiebedarf, der zur Vergrößerung der Gas-Flüssigkeits-Grenzflächen und die Durchmischung benötigt wird stellt einen weiteren Nachteil dieser Methoden dar.

Als weiterer Nachteil kann die kontinuierliche Aufkonzentration von Stoffwechselprodukten in der Fermentationslösung betrachtet werden. Zwar ist es gewünscht, die Produkte in möglichst hohen Konzentrationen zu erhalten, um die Effizienz der anschließenden Produktaufbereitung zu gewährleisten, jedoch haben hohe Produktkonzentrationen gleichzeitig inhibierende Wirkung auf den Produktionsprozess. Wesentliche Aufgaben im Rahmen dieser Ansätze sind es daher zum einen, die Resistenz gegen hohe Produktkonzentrationen zu steigern und zum anderen, das Effizienzmaximum der beiden gegenläufigen Effekte auszuloten.

Bezüglich der niedrigen Populationsdichten führen die verhältnismäßig geringen Konzentrationen der Biokatalysatoren in Kombination mit geringen Konzentrationen von Eduktgasen zu langen mittleren Wegstrecken im Rahmen des Massentransfers. Zwar existieren Mittel die Konzentrationen der Biokatalysatoren zu erhöhen, jedoch führt der einhergehende Anstieg der Viskosität des Mediums oder das Auftreten von Agglomeraten gleichzeitig zu einer Herabsetzung der Diffusionsgeschwindigkeiten im Massentransfer. Durch die geringe Konzentration der Biokatalysatoren werden aber nicht nur die Diffusionsgeschwindigkeiten und somit der Massentransfer herabgesetzt, das einhergehende hohe erforderliche Reaktorvolumen stellt durch hohe Investitionskosten und hohe Betriebskosten für Heizung und Durchmischung wesentliche Faktoren in Hinblick auf die Wirtschaftlichkeit des Prozesses dar.

Der zweite Verfahrensansatz basiert auf der Immobilisierung von Biomasse in Form eines Biofilmes auf einem Festkörper, der mit der reaktiven Gasphase in Kontakt ist. Die fermentative Umsetzung findet in dem Biofilm statt. Durch die Immobilisierung von Biokatalysatoren wird die Problematik der geringen Konzentration von Biokatalysatoren weitestgehend umgangen. Die Biofilme auf den Aufwuchsflächen enthalten die Biokatalysatoren in großen Mengen und mit erheblicher Dichte. Ein dünner Flüssigkeitsfilm bestehend aus Nährmedium versorgt den Biofilm mit essentiellen Substanzen und Wasser, stellt aber zugleich durch seine geringe Dicke nur einen geringen Diffusionswiderstand für den Massentransfer der Eduktgase dar, die sich in großer Nähe in der den Biofilm umgebenden Gasphase befinden. Der geringe Wasseranteil, der für den Betrieb entsprechender Verfahren notwendig ist, stellt einen weiteren Vorteil insbesondere im Zuge der anschließenden Produktaufbereitung dar.

Anwendungsbeispiele sind Rieselbettreaktoren (wie der *immobilized cell reactor* (ICR), *packed bed reactors* oder *trickle bed reactors* (TBR)) und Membranreaktoren (wie der *hollow fiber membrane reactor*). Diese Reaktorkonfigurationen werden derart ausgestaltet, dass möglichst große Gas-Flüssigkeitsgrenzflächen erhalten werden, um den Massetransfer durch Anwendung von Druck zu erhöhen, oder aber einfach einen möglichst einfachen, energiearmen Betrieb zu erlauben. Beispielhaft wird ein Reaktor basierend auf der Immobilisierung der Biomasse in der internationalen Anmeldung WO 2008/154301 A1 beschrieben, in welcher sich die Biomasse in einem Biofilm auf einer Membran befindet, grenzend an ein flüssiges Medium, während auf der anderen Seite der Membran eine Gasphase befindlich ist, in das die umzusetzenden Gase zugefügt werden. Zusätzlich können hier die umzusetzenden Gase auch direkt in das flüssige Medium gegeben werden, so dass die Gase von beiden Seiten der Membran an die Biomasse herangeführt werden.

Die Vorteile dieses Verfahrensansatzes liegen in der hohen Populationsdichte und der hohen Massetransferkoeffizienten, die durch Immobilisierung und große Gas-Flüssigkeitsgrenzflächen erreicht werden.

Nachteile hingegen liegen hier in vergleichsweise hohen Betriebskosten, da häufig inhibierende Prozesse hervorgerufen durch Biofouling abgestorbener Biomasse auftreten. Dies folgt daraus, dass hier keine Möglichkeit besteht, die abgestorbene Biomasse aus dem Prozess zu entfernen. Und so bilden sich im Zuge längerer Betriebszeiten neue Biodiversitäten im Biofilm aus, die zu erheblichen Prozessstörungen bzw. Verlusten im Prozess und somit zu hohen Betriebs- und Instandhaltungskosten führen.

Aufgabe der Erfindung:

Aufgabe der vorliegenden Erfindung ist es, alternative Verfahren und Vorrichtungen zur Umsetzung von Gasen bereitzustellen, bei denen zumindest einige der oben genannten Probleme möglichst reduziert werden.

Erfindungsgemäße Lösung

Die vorliegende Erfindung reduziert überraschenderweise die beschriebenen Probleme durch Anwendung eines alternativen Verfahrens, das im Folgenden kurz beschreiben wird.

Die vorliegende Erfindung betrifft generell ein Verfahren, in dem der Massentransfer der Eduktgase aus der Gasphase zu dem Biokatalysator in Vergleich zu bestehenden Verfahren erheblich begünstigt wird, wobei der Biokatalysator in der die Eduktgase enthaltenden Gasphase in Form eines Aerosols oder auch Bioaerosols vorliegt. Dabei kann das Bioaerosol z. B. durch Verwendung von nachstehend näher beschriebenen Zweistoffdüsen erzeugt werden, oder auf andere Weise hergestellt werden. Allgemein gesprochen wird vorliegend ein Verfahren zur Herstellung biotechnologischer Produkte durch Gasfermentation durch Aerosolbildung respektive Vernebelung oder Tröpfchenbildung der aktive Biomasse enthaltenden Fermentationslösung in einem mit reaktiven Eduktgasen gefüllten Raum offenbart.

Im Gegensatz zu oben erwähnten Ansätzen der mikrobiologischen Umsetzung von Eduktgasen wird in dem beschriebenen Verfahren die aktive Biomasse zusammen mit dem wässrigen Medium als Aerosol oder Nebel in feinen Tröpfchen in den mit den reaktiven Gasen gefüllten Reaktor eingebracht. Vorteile bei diesem Verfahren ergeben sich aus der einfachen Handhabung, geringem Energieeintrag, der großen Gas/Flüssig Grenzflächen, der einfachen Separierbarkeit inaktiver Biomasse, der großen Teilchengeschwindigkeiten und der resultierenden großen Massetransferkoeffizienten.

Biokatalytische Produktionsverfahren auf der Basis von Substratgasen lassen sich allgemein in die drei Verfahrensschritte des Massentransfers, der Synthese und der Produktaufbereitung unterteilen, die jeweils unterschiedlichen Anforderungen unterliegen.

*Massentransfer*

Der Transfer der Eduktgase aus der Gasphase zum Biokatalysator wird als Massentransfer bezeichnet. Die Begriffe Biokatalysator und aktive Biomasse werden, soweit nicht anders explizit vermerkt, für die Zwecke der vorliegenden Beschreibung austauschbar füreinander eingesetzt und beziehen sich generell auf enzymartig wirkende Mikrostrukturen, die natürlicher Abstammung sind, bzw. synthetisch in Anlehnung an solche hergestellt wurden und umfassen Mikroorganismen, Zellen und Enzyme.

Beschreibung des Massentransfers in Formeln:

$$\frac{-\mathrm{dN}}{\mathrm{dt} * \mathrm{V}_R} = k_L * a * (C^* - C_L)$$

dN=Teilchenanzahl

$V_R$ ist das benetzte Volumen des Reaktors, also das Volumen um die aktive Biomasse, und es ist die Summe aus Gasvolumen und Flüssigkeitsvolumen. Die Begriffe Raum, Reaktorraum und Reaktor werden für die Zwecke dieser Beschreibung, falls nicht explizit anders vermerkt, austauschbar füreinander eingesetzt.

dt= Zeit

$k_L$ = Massentransferkoeffizient der Flüssigkeitskomponente - ist eine intrinsische Eigenschaft von Gas und Lösung, daher schwer zu ändern - in den konventionellen Verfahren existiert nahezu keine Einflussnahmemöglichkeit. In der vorliegenden Erfindung gilt dies vorteilhafterweise nicht. Hier ist eine Einflussnahme durch Variation der Flüssigkeitshülle um den Biokatalysator möglich. Dies kann gemäß der vorliegenden Erfindung auf unterschiedlichen Wegen erreicht werden.

Zunächst kann eine Einflussnahme über die Einrichtung zur Aerosolerzeugung, wie z.B. einen Zerstäuber erreicht werden. Wird als Zerstäuber beispielsweise eine Düse eingesetzt, so beeinflussen die Steuerung der Durchflussmengen und die Wahl der Düse in Abhängigkeit der Viskosität der Lösung die Partikelgröße und somit die Dicke der Grenzschicht, bzw. der Fluidhülle um den Biokatalysator. Ferner beeinflusst der in der Düse erzeugte Druck die Geschwindigkeit der Gasmoleküle, die bei Austritt aus der Düse zum Biokatalysator (aktiver Biomasse) gelangen. Durch Erhöhung des Drucks und/oder Verkleinerung der Düsenaustrittsöffnung kann die Schichtdicke des Fluids um die aktive Biomasse verkleinert und somit der Gastransfer durch diese Hülle zu der Biomasse beschleunigt werden. Ferner hat das Aerosol an sich einen langfristigen Effekt. Durch turbulente Strömung erzeugter Druck beeinflusst die Geschwindigkeit der Gasmoleküle bei Eintritt in die Grenzschicht und in der Grenzschicht zur Biomasse. Die Dauer des Effektes ist hier abhängig von der Lebenszeit des Aerosols.

$$\text{Driving Force} = (C^{*}\text{-}C_{L})$$

$C_L$ = Gaskonzentration in Lösung - Unter Massentransfer limitierten Bedingungen (== Gesamtreaktion wird durch Geschwindigkeit des Massentransfers bestimmt- wird allgemein angenommen) ist $C_L$=0

C* beschreibt die Gaslöslichkeit und ist proportional zu Partialdrücken der Gase- kann durch Druck erhöht werden.

In konventionellen Methoden erfordert der Druckaufbau viel Energie.

Gemäß den Verfahren der vorliegenden Erfindung ist eine energiearme Ausübung von Druck durch zwei Effekte möglich:

Im Zerstäuber, z.B. in einer Düse kann ein kurzfristiger Effekt dadurch erreicht werden, dass C* auf C** durch Druckerzeugung in der Düse.

Im Aerosol kann ein langfristiger Effekt erreicht werden. Hierzu wird C* auf C*** erhöht durch Aerosolgeschwindigkeit, die durch turbulente Strömung erzeugt werden. Die Dauer des Effektes ist wiederum abhängig von der Lebenszeit des Aerosols.

$$a = \text{spezifische Grenzfläche für Massentransfer (Gas-Flüssigkeits-(Gas-Partikel-) Grenzfläche)}$$

Wie schon erwähnt, gibt es in den konventionellen Verfahren im Wesentlichen zwei Ansätze zur Erhöhung des Massentransfers von Eduktgasen zu aktivem Biomaterial. Zum einen werden in Flüssigkeit Gasblasen erzeugt, zum anderen werden Biofilme auf Aufwuchsflächen angebracht, um so die aktive Oberfläche zu steigern.

Erfindungsgemäß werden im Wesentlichen folgende Ansätze verfolgt:

- In der Düse ein kurzfristiger Effekt: Erhöhung von a durch Zerkleinerung der Flüssigkeitsbereiche bereits bei Austritt aus Düse
- Im Aerosol ein langfristiger Effekt: Erhöhung von a durch fein verteilte Partikel in gasförmigen Medium - die Dauer des Effektes ist hier abhängig von der Lebenszeit des Aerosols

  ◦ $a = NP_B * 4 * \pi * \overline{r_P}$
  ◦ $NP_B$=Anzahl der Partikel im Aerosol, in denen Biokatalysatoren enthalten sind

    ▪ Ist abhängig von Zellkonzentration und Verklumpungsgrad in Fermentationslösung.
    ▪ Ist ferner abhängig von der mittleren generierten Partikelanzahl (== Funktion(Partikelgröße)) pro Volumenstrom des Biokatalysator-Stromes

  ◦ $\overline{r_P}$= mittlerer Partikel Radius
  ◦ NP= mittlere Gesamtanzahl der generierten Partikel
  ◦ $\frac{NP}{dt} = \frac{F_L}{V_P}$
  ◦ $F_L$ = Volumenstrom der Fermentationslösung
  ◦ $V_P = \frac{4}{3} * \pi * \overline{r_P}^3$=Partikelvolumen

Biokatalysatoren weisen eine unterschiedliche Empfindlichkeit gegen Stärke und Dauer ausübender Drücke und Scherkräfte auf. Drücke im Zerstäuber und Aerosolturbulenz müssen daher auf die jeweils eingesetzten Biokatalysatoren angepasst werden. Es besteht die Möglichkeit, durch Variation der umgebenden Flüssigkeitshülle in Zusammensetzung (erhöhte Lösungsviskosität, Einbettung der Biokatalysatoren in viskose oder feste Komponenten) oder Form (Dicke der Flüssigkeitshülle durch Steuerung der Partikelgrösse im Aerosol) weiteren Einfluss zu nehmen und einen Schutz für Biokatalysatoren zu bieten. Dieser wird allerdings die Massentransfereffizienz herabsetzen, sollte daher so gering wie möglich gehalten werden. Um die Verweildauer in der Aerosolform zu verlängern, bzw. möglichst lange zu erhalten kann auch die Form des Reaktors gestaltet werden. In einer bevorzugten Ausführungsform werden Reaktoren eingesetzt, die in Richtung des abgegebenen Aerosolstrahls verlängert sind, d.h. rohrförmige Reaktoren mit dem Aerosolstrahl an einem Ende des Reaktors. Die beladene aktive Biomasse bzw. die im Reaktor hergestellten Produkte werden dann bevorzugt am gegenüberliegenden Ende des Reaktors ausgeleitet.

Damit die biokatalytische Reaktion im Anschluss stattfinden kann, muss durch den Massetransfer die Versorgung des Biokatalysators mit Eduktgasen in ausreichender Menge in einem, der beabsichtigten Reaktion entsprechenden Verhältnis bewerkstelligt werden. Die Funktionsfähigkeit des Biokatalysators darf durch den Massentransfer nicht beeinträchtigt werden.

Weitere Ziele im Rahmen des Massentransfers, die der Effizienzsteigerung des erfindungsgemäßen Verfahrens dienen erfordern, dass der Transfer in möglichst kurzer Zeit stattfindet, der Transfer zu ein und demselben Biokatalysator wiederkehrend stattfindet und der zeitliche Abstand zwischen zwei Transferaktionen möglichst kurz ist. Weiterhin sollte der Transferprozess wenig Raumvolumen einnehmen, der Energieeintrag möglichst gering gehalten werden und der technische Aufwand für den Transfer möglichst gering gehalten werden.

*Syntheseprozess*

Die biokatalytische Synthese beschreibt die Umwandlung der Eduktgase zu dem Produkt oder den Produkten. Damit dieser Prozess stattfinden kann, muss der richtige Biokatalysator vorliegen und aktiviert sein. Außerdem müssen die Umgebungsvariablen und Prozessparameter wie Druck, Temperatur, pH-Wert, Redox-Potential und Nährstoffverhältnisse die geeigneten Bedingungen erfüllen. Inhibitoren dürfen nicht, oder nur in geringen Mengen vorliegen. Durch stetige Zufuhr von Eduktgasen, bzw. Eduktgas-Aerosol-Gemisch sollte die durch die Reaktion stattfindende Reduktion des Gasvolumens ausgeglichen werden und ein leichter Überdruck in dem Reaktor erhalten werden.

Soll die Synthese optimiert werden, so muss außerdem sichergestellt sein, dass die Anzahl der Umwandlungsprozesse pro Biokatalysator maximiert wird, der Biokatalysator also fortwährend reaktiviert wird und immer wieder mit Edukten versorgt wird, die Umwandlung der Eduktgase möglichst vollständig erfolgt, Nebenprodukte in Anzahl und Menge minimiert werden, und der Aufwand für den Syntheseprozess minimal gehalten wird. Ansätze die zum Erreichen dieser Ziele benötigt werden sind sowohl im Bereich der Mikrobiologie, als auch in der (Bio-)Verfahrenstechnik zu finden.

*Produktaufbereitung*

Im Rahmen der Produktaufbereitung wird das oder die Zielprodukte von Nebenprodukten abgetrennt und für anschließende Verwendung aufbereitet. Zu den einzelnen Schritten gehören Abtrennung des oder der Zielprodukte, gegebenenfalls eine weitere Umsetzung, eine Aufkonzentration und die Aufbereitung. Der Prozess soll möglichst unter minimalem Energieaufwand, mit hoher Zuverlässigkeit stattfinden und unter minimalen Anlagen- und Komplexitätsaufwand erfolgen. Ein im Reaktor integrierter Abtrennungs- und Aufkonzentrationsprozess ist daher von hohem Interesse.

Der extrem hohe Bedarf an Gasen mit verhältnismäßig geringen Löslichkeiten macht den Massentransfer zum bedeutendsten Schritt biokatalytischer Produktionsverfahren auf der Basis gasförmiger Substrate. Und nach wie vor gilt aufgrund der unzureichenden Lösungsansätze dieser Schritt als Flaschenhals entsprechender Verfahren.

Allgemeine Herausforderungen, um dieses Problem zu umgehen, sind die Vergrößerung von Gas-Flüssigkeits-Grenzflächen und die Erhöhung von Diffusionsgeschwindigkeiten der Gase durch beruhigte Flüssigkeitszonen um eine Verschiebung des Gleichgewichtes zu erreichen.

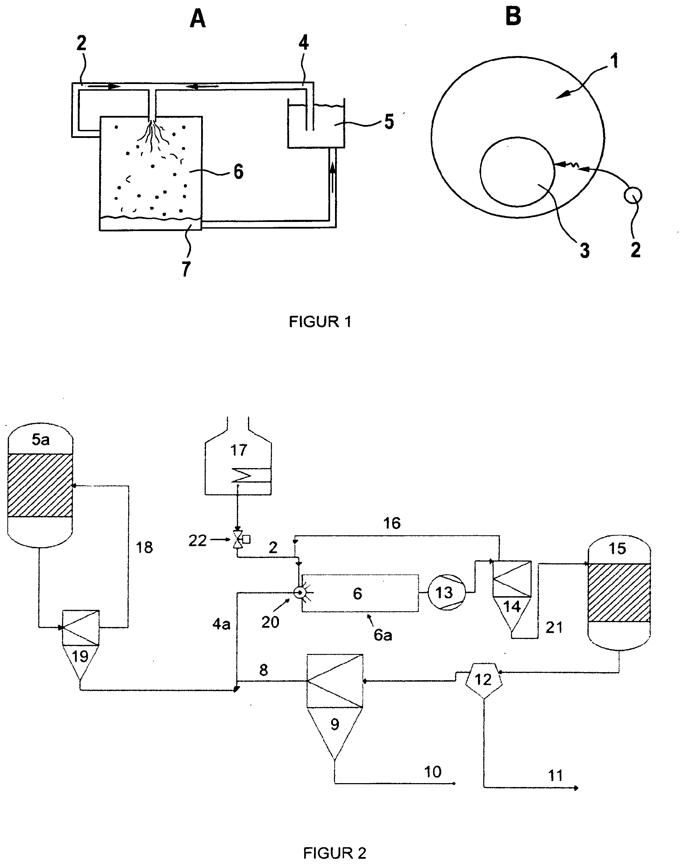

Basierend auf dem oben genannten generellen Prinzip sind verschiedene Ausführungsformen des erfindungsgemäßen Verfahrens denkbar. Beispielsweise wird durch die vorliegende Erfindung auch eine Vorrichtung und ein Verfahren bereitgestellt, in dem der Massetransfer der Eduktgase zu dem Biokatalysator durch Verwendung von Zweistoffdüsen (oder ähnlicher Einrichtungen) erheblich beschleunigt wird. Dabei werden in der Zweistoffdüse zwei Stoffströme zusammengeführt, wobei der eine Stoffstrom die Eduktgase enthält und der andere Stoffstrom den Biokatalysator enthält (siehe Figur 1). Der Biokatalysator liegt dabei in einer bevorzugten Ausführungsform im flüssigem Medium, in einer anderen Ausführungsform liegt er dagegen vorwiegend in fester Form als Teil eines Pulvers vor, welches Pulver in einer Ausführungsform im befeuchteten Zustand vorliegt.

Ein Vorteil der vorliegenden Erfindung ist die Erhöhung des Massentransfers durch gezieltes und beschleunigtes Zusammentreffen von Eduktgasen mit Biokatalysatoren. Dieses wird durch verschiedene hier beschriebenen Maßnahmen erreicht, wie durch Verwendung von Zweistoffdüsen, in denen der eine Stoff aus einem Gasstrom besteht, der die Eduktgase enthält und der andere Strom den Biokatalysator in meist flüssiger Form enthält. Der Biokatalysator kann im trockenen Zustand beispielsweise als Pulver, in angefeuchtetem Zustand oder gelöst in einem flüssigen Medium vorliegen. In einer Ausführungsform enthält dieser Strom den Biokatalysator der an einen Feststoff gebunden ist. Beispiele hierfür sind biologisch inerte Feststoffe, wie beispielsweise Partikel aus organischen Materialien umfassend aber nicht beschränkt auf Polymere, Polysaccharide (beispielsweise vernetzte Polysaccharide wie Agarose, Dextran, Zellulose, Stärke usw.), Proteine und synthetische Polymere (z.B. Polystyren, Polyacrylamid, Homopolymere und Copolymere der Derivate von Acrylat und Methacrylat), inorganische Partikel umfassend Partikel aus Silikonen oder Glas, und Metallpartikel, wie Partikel aus Gold, Selen, Platin und anderen Metallen.

Durch Abstimmung der Ströme in der Zweistoffdüse kann die Zielkonzentration der Gase in dem den Biokatalysator umfassenden Medium gezielt beeinflusst werden Durch Anwendung von hohem Druck und erhöhten Relativgeschwindigkeiten zwischen Eduktgasen und dem den Biokatalysator umfassenden Medium im Inneren der Düse ist der Übergang der gasförmigen Edukte in die Flüssigkeit vorteilhafterweise nicht durch die geringen Gaslöslichkeiten bei Normaldruck bestimmt. Eine Verwendung viskoser Flüssigkeiten mit erhöhter Zellkonzentration ist durch Verwendung spezieller Düsen möglich. Düsen haben generell die Eigenschaft, dass Druck in kinetische Energie umgewandelt wird. Druck also ungerichtete kinetische Energie wird abgebaut, während die Austrittsgeschwindigkeit des Gemischs aus der Düse erhöht wird.

Die Wahl der Düsen und der Gasströme kann ferner oder optional auch auf die Sensitivität der jeweiligen Biokatalysatoren gegen Scherkräfte abgestimmt werden.

Alternativ oder zusätzlich können die erfindungsgemäß eingesetzten Biokatalysatoren vor zu hohen Scherkräften geschützt werden, indem sie von viskosen Medien oder auch Feststoffen umgeben sind.

Das durch die Zweistoffdüse erzeugte Medium kann entweder in den gasenthaltenden Raum zur Bildung eines Aerosols gesprüht werden, oder aber in Flüssigkeit oder gegen eine Abscheidevorrichtung gesprüht werden, um zur direkten weiteren Verwendung, beispielsweise der Produkteabtrennung überzugehen.

All diese Maßnahmen führen für sich bzw. zusammen zu einer erhöhten Gas-Flüssigkeitsoberfläche und einem geringen Flüssigkeitsvolumen um den Biokatalysator. Dies bewirkt reduzierte Weglängen der Eduktgase zum Biokatalysator, wodurch das Mengenverhältnis vom Gas zum Biokatalysator erhöht wird.

Die so hergestellten Flüssigkeitstropfen in dem Aerosol wirken wie kleine Reaktoren für sich, die jeweils eigenen Bestimmungen unterliegen und die im stetigen Austausch mit der Umgebung liegen, wodurch ein ständiger Ausgleich von Ungleichgewichten während Bestehen des Aerosols ermöglicht wird.

Ebenfalls können durch die geringe Dicke der Fluidschicht um die Partikel der aktiven Biomasse, die dort erzeugten Stoffwechselprodukte das Einflussgebiet des Biokatalysators durch Diffusion einfach verlassen und in die Gasphase übergehen, was die Gewinnung des oder der Produkte erleichtert.

Wie schon oben erwähnt, ist eine Verwendung viskoser Flüssigkeiten mit erhöhter Zellkonzentration durch Verwendung spezieller Düsen möglich, dadurch wird die Anzahl der Mikroreaktoren erhöht, bzw. das Verhältnis wirksamer Flüssigkeitstropfen mit Biokatalysator zu unwirksamen Flüssigkeitstropfen ohne Biokatalysator begünstigt.

In einer Ausführungsform wird das Aerosol nach dessen Niederschlag nach evtl. Aufbereitung wieder verwendet werden (Kreislauf des Aerosolmediums). Hierzu wird in einer bevorzugten Ausführungsform eine Kondensationsvorrichtung verwendet, die das Niederschlagen des Aerosols vereinfacht. Bevorzugt wird hierzu eine kalte Oberfläche verwendet, die in dem Reaktorraum angebracht wird, oder eine der Wände des Reaktors, die entsprechend gekühlt wird, oder der Boden des Reaktors bzw. andere übliche Einrichtungen zur Aerosolabscheidung wie zum Beispiel Prallabscheider oder Zyklone. In einer Ausführungsform wird der Reaktorboden dementsprechend in Trichterform gestaltet, um den Abfluss des niedergeschlagenen Aerosols zu erleichtern.

Kreislauf des Mediums hat Vorteil der leichten Prozessregulierung durch: Nährstoffausgleich, Support durch Additive, Abtrennung abgestorbener Biomasse, Messmethoden zur Überwachung der Prozessparameter können in den Kreislauf installiert werden.

Nicht verwertete Gasströme werden gemäß einer Ausführungsform erneut in den Prozess eingeführt. Hierzu wird ein Kreislauf der Gasströme oder eine Hintereinanderschaltung von Reaktoren eingesetzt.

Die Dicke der Flüssigkeitshülle wird in einer Ausführungsform durch Wahl der Düsen und Stoffströme gesteuert. Dadurch kann eine Abstimmung der Umgebungsvariablen an die Bedürfnisse des Biokatalysators erfolgen.

Prozessoptimierung

Die oben beschriebenen Verfahrens- bzw. Prozessschritte können optional durch verschiedene im Nachfolgenden beschriebene Maßnahmen optimiert werden. So kann durch Erzeugung turbulenter Strömung im Aerosol die Diffusionsgeschwindigkeit des Gases aus der Gasphase in den Flüssigkeitsfilm um den Biokatalysator weiter beschleunigt werden.

Anstatt eines singulären Reaktors kann Reihen oder Parallelschaltung von Reaktoren angewendet werden, um die Effizienz des Prozesses zu steigern.

Übliche Verfahren oder Mittel, wie beispielsweise die Steigerung der Reaktionstemperatur können zur Steigerung der Aktivität von erfindungsgemäßen Biokatalysatoren verwendet werden.

In einer Ausführungsform stellt bzw. stellen das oder die erfindungsgemäß hergestellten Produkte lediglich Zwischenprodukte dar, die zu Zielprodukten umgesetzt werden müssen. Diese anschließende Reaktion synthetisierter Produkte zu Zielprodukten wird im Reaktor oder auch im Anschluss, außerhalb des Reaktors durchgeführt. Bei der Durchführung der Reaktion im Reaktor werden gegebenenfalls notwendige weitere Katalysatoren oder Edukte im Prozess integriert.

In einer weiteren Ausführungsform werden weiterhin Messvorrichtungen installiert zur Erfassung von Prozessparametern, wie Temperatur, pH-Wert, Druck, Redox-potential und Nährstoffverhältnisse. Bevorzugt sind diese Messvorrichtungen mit entsprechenden Regeleinrichtungen verbunden um besagte Parameter, z.B. bei Abweichungen von den gewünschten Größen zu verändern.

Weiterhin werden in noch einer Ausführungsform Lichtquellen installiert, die später genauer beschrieben sind, wobei genutzt wird, dass die großen Gas-Flüssigkeitsoberflächen des Aerosols gleichbedeutend sind mit großer Absorption von Licht und somit mit der Möglichkeit große Mengen Biomasse herzustellen.

Abtrennung der Produkte

Die Abtrennung der Produkte kann entweder direkt im Prozess erfolgen, im Anschluss aus dem niedergeschlagenen Medium oder beides.

Produkte können aus der Gasphase durch Membrantechniken, im Gegenstromverfahren oder andere Techniken einfach bereits im Prozess abgetrennt werden.

Durch Abtrennung der Produkte im Prozess wird das Gleichgewicht günstig beeinflusst und Hemmstoffe können entfernt werden. In bevorzugten Ausführungsformen des erfindungsgemäßen Verfahrens und der erfindungsgemäßen Vorrichtung werden daher durch geeignete Positionierung von Leitungen und Abscheidern die Prozessprodukte möglichst schnell nach Verlassen des Reaktors, zum Teil auch schon aus dem Reaktorraum selbst abgetrennt.

Durch den erhöhten Massentransfer kann der Wasseranteil im erfindungsgemäßen Verfahren im Vergleich zu anderen Verfahren erheblich reduziert werden. Dies wirkt sich positiv auf eine anschließende Produktaufbereitung aus.

Detaillierte Beschreibung der Erfindung

Wie schon oben dargelegt, betrifft die vorliegende Erfindung generell auf ein Verfahren zur mikrobiologischen Umsetzung von reaktiven Eduktgasen zu einem oder mehreren Produkten, dadurch gekennzeichnet, dass eine aktive Biomasse umfassende, im Wesentlichen daraus bestehende oder daraus bestehende Zusammensetzung als Aerosol in einen mit den reaktiven Eduktgasen gefüllten Raum eingebracht wird.

Der Ausdruck "in einen mit reaktiven Eduktgasen gefüllten Raum" bezieht sich darauf, dass gemäß dem Verfahren der vorliegenden Erfindung die umzusetzenden Gase nicht in das flüssige Medium eingeführt werden, in welchem sich bevorzugt die aktive Biomasse befindet, welches Vorgehen in den oben beschriebenen Verfahren und Vorrichtungen nach Stand der Technik eingesetzt wird, sondern, dass die umzusetzenden Gase in die Gasphase eingeführt werden, die sich im Reaktor befindet, bzw. in dem Raum, in dem die Umsetzungsreaktionen stattfinden. Die aktive Biomasse wird dabei in den Reaktor, bzw. den Raum als Aerosol eingeführt. Wie der Wortlaut nahelegt, kann das Aerosol durch geeignete Einrichtungen zur Aerosolerzeugung, wie z.B. Düsen während des Einbringens in den Raum entstehen, oder bereits vorher erzeugt worden sein (siehe auch Fig. 1A). Alternativ wird gemäß einer Ausführungsform das Aerosol zunächst erzeugt und dann in dieser Form in den Raum eingebracht. Dies kann beispielsweise derart erreicht werden, indem der Aerosolerzeuger in einem Vorraum vor dem Reaktor untergebracht und das dort entstehende Aerosol nach Erzeugung in den Reaktorraum eingebracht wird.

Die aktive Biomasse kann in dem erfindungsgemäßen Verfahren bei Einbringen in den Reaktor in einem geeigneten Medium vorliegen oder in Pulverform eingesetzt werden. Liegt die aktive Biomasse in Pulverform vor, so ist dieses bevorzugt angefeuchtet. Bevorzugt liegt die aktive Biomasse jedoch in einem Medium vor, wobei das Medium bevorzugt so ausgewählt wird, dass die Biomasse darin gelöst werden kann. In einer bevorzugten Ausführungsform des erfindungsgemäßen Verfahrens enthält die Zusammensetzung daher ferner ein Medium. Da die bevorzugten Vertreter der aktiven Biomasse, wie später noch genauer definiert wird, löslich in wässrigen Medien sind, wird gemäß der vorliegenden bevorzugt ein wässriges Medium eingesetzt, d.h. in einer bevorzugten Ausführungsform ist das eingesetzte Medium ein wässriges Medium. In einer Ausführungsform werden auch alternative Medien zur Aufnahme der aktiven Biomasse eingesetzt, umfassend aber nicht beschränkt auf nicht-wässrige Medien, wie hydrophobe, beispielsweise ölhaltige oder aus Ölen bestehende Medien, kohlenwasserstoffhaltige Medien oder aus Alkoholen bestehende Medien. In einigen bevorzugten Ausführungsformen werden die Medien auch bezüglich ihrer Viskosität variiert, wie oben schon beschrieben wurde, um beispielsweise die darin enthaltene aktive Biomasse in dem Verfahren zu schützen.

Die Zuführung der Eduktgase in die Gasphase des Reaktionsraums kann getrennt von der Biomasse erfolgen, z.B. durch eine getrennte Einspeiseeinrichtung oder eine getrennte Leitung. Bevorzugt wird die aktive Biomasse zusammen mit den reaktiven Eduktgasen in den Reaktorraum eingeführt. Somit stellt die vorliegende Erfindung in einem Aspekt ein Verfahren wie hierin beschrieben zur Verfügung, wobei die Zusammensetzung enthaltend die aktive Biomasse, die aktive Biomasse und ein Medium, wobei das Medium bevorzugt ein wässriges ist, zusammen mit den Eduktgasen als Aerosol in den mit den reaktiven Eduktgasen gefüllten Raum eingebracht wird. Zur Aerosolbildung kann ein weiteres Gas dienen. Da dies jedoch die Konzentration der umzusetzenden reaktiven Eduktgase in der Reaktion senken würde, wird bevorzugt umzusetzendes Eduktgas zur Aerosolerzeugung eingesetzt. Damit liegt dieses dann im Reaktionsraum als Bioaerosol vor, also eine Dispersion aus dem Eduktgas, der aktiven Biomasse und, soweit eingesetzt, dem jeweiligen Medium. Werden Zerstäuber, wie Düsen zur Aerosolerzeugung eingesetzt, so wird das jeweils eingesetzte Eduktgas bevorzugt mit einem höheren Strömungsfluss eingesetzt als die Zusammensetzung enthaltend die aktive Biomasse, bzw. die Biomasse und das jeweilige Medium. Bevorzugt wird das Verhältnis der Strömungsflüsse dabei so ausgewählt, dass das Eduktgas eine zehnfach so hohen oder höheren Strömungsfluss hat als die eingesetzte Zusammensetzung. In einer bevorzugten Ausführungsform enthält die Zusammensetzung neben der Biomasse und gegebenenfalls den weiteren erwähnten Komponenten auch weitere Zuschlagstoffe. Beispiele für solche Zuschlagstoffe sind Naturstoffe wie Vitamine, Enzyme, ausgewählte Inhibitoren oder andere biologisch aktive Substanzen, die Stoffwechselprozesse von Mikroorganismen beeinflussen oder auch Stoffe, die durch ihren pH-Wert oder ihr Redoxpotential die Reaktion begünstigen.

Durch die Aerosolbildung kommt es dazu, dass die aktive Biomasse im Aerosol in feinen Tröpfchen vorliegt, wobei die einzelnen Partikel, oder gegebenenfalls Agglomerate umfassend einige Partikel der jeweils eingesetzten Biomasse von einer Fluidhülle aus dem in der Zusammensetzung befindlichen Medium umgeben sind. Die Anzahl der Biomassepartikel in solchen gegebenenfalls vorhandenen Agglomeraten ist von verschiedenen Faktoren umgeben, u.a. auch von den jeweils eingesetzten Vertretern der aktiven Biomasse und dabei auch von deren Umfang, wie auch von dem Umfang der jeweils erzeugten Aerosoltropfen. In der Regel liegt die Biomassenpartikelanzahl bei etwa $10°$ bis $10^2$ Partikel pro Aerosoltröpfchen, wobei die Partikelanzahl bei Mikroorganismen oder Zellen im niedrigeren, bei Enzymen im höheren Bereich liegen kann. Insbesondere bei Mikroorganismen bzw. Zellen wird die Aerosolerzeugung derart gesteuert, dass bevorzugt mindestens $1 \times 10^{4,}$ oder auch $10^3$ in anderen Ausführungsformen mindestens 100, in noch anderen Ausführungsformen mindestens 50, oder mindestens 10 Mikroorganismen bzw. Zellen pro Aerosoltröpfchen (ca 1 Mikroliter Volumen) vorliegen, in Abhängigkeit von der Größe des Mkroorganismus bzw. Zelle. Hierzu werden bekannte Verfahren eingesetzt, die schon vorhin beschrieben wurden, wie beispielsweise die Wahl geeigneter Öffnungsgröße bei der als Zerstäuber eingesetzten Düse, und/oder eine entsprechende Anpassung des Strömungsflusses des Eduktgases und/oder der Zusammensetzung enthaltend die aktive Biomasse.

Entsprechend wird in einer bevorzugten Ausführungsform das erfindungsgemäße Verfahren bereitgestellt wie hierin beschrieben, wobei die aktive Biomasse von einer Fluidhülle aus einem Film des in der Zusammensetzung enthaltenden Mediums umgeben ist. Die Dicke der Fluidhülle ist entscheidend für den Massetransfer vom Gas zu der aktiven Biomasse. Wie gerade dargelegt, kann die Größe der Aerosoltröpfchen durch geeignete Maßnahmen beeinflusst werden, so dass sie möglichst kleine Ausmaße annimmt. In einer bevorzugten Ausführungsform des Verfahrens liegt dabei die Schichtdicke der Fluidhülle um die aktive Biomasse bei etwa 0,5 $\mu$m bis etwa 200 $\mu$m, bevorzugt bei etwa 1 $\mu$m bis etwa 50 $\mu$m, weiter bevorzugt bei etwa 2 $\mu$m bis etwa 30 $\mu$m, besonders bevorzugt bei etwa 5 $\mu$m bis etwa 20 $\mu$m.

Durch stetige Zufuhr von Eduktgasen, bzw. Eduktgas-Aerosol-Gemisch wird in einer bevorzugten Ausführungsform die durch die Reaktion stattfindende Reduktion des Gasvolumens ausgeglichen und ein leichter Überdruck in dem Reaktor erzeugt und gehalten, wobei unter Überdruck ein Druck von mindestens 1100 mbar, bevorzugt bis zu 2000 mbar verstanden wird.

Bezüglich der eingesetzten aktiven Biomasse ist es wesentlich, dass diese enzymartig wirkende Mikrostrukturen enthält, die zur Umsetzung der eingesetzten reaktiven Eduktgase beitragen können, indem sie beispielsweise entsprechende Zwischenprodukte aus diesen herstellen, bzw. die Umwandlung durchführen oder vollenden können, indem sie das oder die gewünschten Endprodukte aus den eingesetzten reaktiven Eduktgasen bzw. aus den Zwischenprodukten erzeugen. Daher umfasst das erfindungsgemäße Verfahren gemäß der vorliegenden Erfindung aktive Biomasse, wobei die aktive Biomasse umfasst, im Wesentlichen besteht oder besteht aus ein oder mehreren Mikroorganismen, Zellen und/oder Enzymen. Unter Enzymen werden gemäß der vorliegenden Erfindung natürliche Enzyme (also Proteine), naturidentische oder ähnliche Enzyme, teil- oder vollsynthetische Enzyme (Synzyme) verstanden sowie weitere katalytisch aktive Verbindungen wie beispielsweise RNAs (Ribozyme), die alleine oder in Verbindung mit Proteinen katalytisch sind. Die aktive Biomasse kann dabei homogen sein, d.h. aus einem Typus bzw. Art eines Mikroorganismus, einer Zelle oder eines Enzyms bestehen, oder mehrere Typen bzw. Arten von Mikroorganismen, Zellen oder Enzymen enthalten, so dass beispielsweise der eine Typus bzw. Art das umzusetzende Eduktgas in ein Zwischenprodukt umsetzt, welches dann von einem anderen Typus bzw. Art in das Endprodukt umgesetzt wird.

Werden in dem erfindungsgemäßen Verfahren Mikroorganismen oder Zellen eingesetzt, so werden in einer bevorzugten Ausführungsform ein oder mehrere hiervon eingesetzt ausgewählt aus der Gruppe umfassend: acetogene Bakterien, methanotrophe Bakterien, photosynthetisch tätige Mikroorganismen umfassend Algen und phototrophe Bakterien.

Ein bevorzugt eingesetzter Vertreter actetogener Bakterien sind Bakterien der *Clostridien*-Gattung, besonders bevorzugt *Clostridium ljungdahlii.* In einer Ausführungsform werden acetogene Bakterien wie das *Clostridium ljungdahlii* gemäß der vorliegenden Erfindung zur Umsetzung von Synthesegas (Syngas) zu Ethanol und oder Essigsäure eingesetzt.

Die in den erfindungsgemäßen Verfahren eingesetzten Eduktgase stellen einzelne Gase oder Gasgemische dar und entstammen meistens anderen Prozessen, wie z.B. Vergärung oder Pyrolyse von Biomasse, wie sie Beispielsweise bei Abfallbeseitigung oder Abwasserreinigung entstehen. Da diese teils toxisch und teils klimaschädlich sind, gibt es ein allgemeines Bestreben, diese soweit möglich in weniger schädliche und möglichst noch nutzbare Produkte umzusetzen. In einer bevorzugten Ausführungsform wird daher das erfindungsgemäße Verfahren bereitgestellt, wie hierin beschrieben, wobei die reaktiven Eduktgase eines oder mehrere sind aus der Gruppe umfassend Kohlenmonoxid, Kohlendioxid, Sauerstoff, Stickstoff, Wasserstoff, Ammoniak, Schwefelwasserstoff, Stickoxide, Methan, Synthesegas, Biogas, Formaldehyd und Kohlenwasserstoffe.

Der Begriff Biogas wird hierbei zusammenfassend für Gasgemische verwendet, die unter anoxischen Bedingungen durch Mikroorganismen aus biotischen Stoffen gebildet werden und umfasst Klärgas, Faulgas und Deponiegas. Der Begriff Synthesegas oder Syngas bezieht sich auf ein Gasgemisch, welches beispielsweise bei Pyrolyse ligninhaltiger Substrate entsteht und primär eine Mischung aus Kohlenmonoxid, Wasserstoff und Kohlendioxid mit anderen Komponenten, wie Methan, Stickstoff, Ammoniak, Schwefelwasserstoff und anderen Spurengasen darstellt.

Die reaktiven Eduktgase werden zu einem oder mehreren unterschiedlichen Produkten umgesetzt, die zum Teil als Ausgangsprodukte und zum Teil als Energieträger dienen. In einer bevorzugten Ausführungsform wird das erfindungsgemäße Verfahren bereitgestellt, wie hierin beschrieben, wobei ein oder mehrere Produkte ausgewählt sind aus der Gruppe umfassend: Alkohole wie Ethanol, Methanol, Butanol, Kohlenwasserstoffe wie Methan, Wasserstoff, Carbonsäuren wie Essigsäure oder deren Ester, Carbonylverbindungen wie Aceton oder Formaldehyd, Stickstoff-Wasserstoff-Verbindungen wie Ammoniak, Biomasse oder relevante Bestandteile davon.

Der Begriff Biomasse bezieht sich hierbei auf Vermehrung von Mikroorganismen durch das Erfindungsgemäße Verfahren, vorzugsweise von Mikroorganismen, welche die umzusetzenden Eduktgase verstoffwechseln und somit zum Aufbau neuer Biomasse nutzen können. Ein Beispiel hierfür sind photosynthetisch tätige Mikroorganismen, wie z.B. Algen oder phototrophe Bakterien, die Kohlenstoffdioxid zusammen mit Licht als Energielieferant nutzen können, acetogene Mikroorganismen, die Kohlendioxid oder Kohlenmonoxid und $H_2$ verstoffwechseln, oder auch methanotrophe Bakterien, die Methan verstoffwechseln. Der Begriff Biomasse umfasst in diesem Zusammenhang die Mikroorganismen an sich und auch alle von ihnen hergestellten Produkte, bzw. deren Bestandteile, wie Aminosäuren, Proteine, Zucker, Vitamine usw. Neben Kohlenstoffquellen wie Kohlendioxid benötigen photosynthetisch aktive Organismen wie schon angesprochen Licht. Soll Biomasse das herzustellende Produkt sein, werden die photosynthetisch aktiven Mikroorganismen während des Verfahrens daher Licht ausgesetzt, wobei Lichtquellen wie Tageslichtfenster oder Lampen eingesetzt werden, wie weiter unten in Bezug auf die erfindungsgemäß bereitgestellte Vorrichtung genauer beschrieben wird. Damit das Licht bis zu den Mikroorganismen durchdringen kann umfassen in einer Ausführungsform die Reaktorwände Bereiche aus lichtdurchlässigen Materialien oder bestehen aus solchen.

In einem weiteren Aspekt betrifft die vorliegende Erfindung ferner eine Vorrichtung zur mikrobiologischen Umsetzung reaktiver Eduktgase zu einem oder mehreren Produkten, umfassend

(i) einen Reaktor;
(ii) eine Einrichtung zur Zuführung von aktiver Biomasse in den Reaktor;
(iii) eine Einrichtung zur Zuführung von mindestens einem reaktiven Eduktgas in den Reaktor; und
(iv) eine Einrichtung zur Aerosolerzeugung;

derart eingerichtet, dass die aktive Biomasse durch die Einrichtung zur Aerosolerzeugung als Aerosol in den Reaktor eingebracht wird und wobei das mindestens eine reaktive Eduktgas und die aktive Biomasse in die Gasphase im Reaktor eingeführt werden.

Die beiden Möglichkeiten, wie das Aerosol in den Reaktor eingebracht werden kann, sind weiter oben beschrieben worden. In einer Ausführungsform wird daher eine Vorrichtung bereitgestellt, wobei die Vorrichtung zur Aerosolerzeugung (Aerosolerzeuger) in einem Vorraum zum Reaktor angebracht ist und dort das Aerosol erzeugt.

Da, wie schon oben dargelegt, das umzusetzenden Eduktgas bevorzugt als Triebkraft zur Aerosolerzeugung eingesetzt wird, bzw. zusammen mit der aktiven Biomasse als Bioaerosol in den Reaktor eingebracht wird, wird die erfindungsgemäße Vorrichtung wie hierin beschrieben bevorzugt derart aufgebaut, dass die Einrichtung zur Zuführung von aktiver Biomasse in den Reaktor und die Einrichtung zur Zuführung von mindestens einem reaktiven Eduktgas in den Reaktor in der Einrichtung zur Aerosolerzeugung münden und diese derart eingerichtet ist, die zugeleiteten Substanzen als Aerosol in die Gasphase im Reaktor einzubringen. Ferner sind in einer bevorzugten Ausführungsform die Einrichtungen zur Zuführung von aktiver Biomasse in den Reaktor und zur Zuführung von mindestens einem Reaktiven Eduktgas in den Reaktor als Leitungen ausgestaltet, die dann entsprechend im Aerosolerzeuger münden. Ein Beispiel für Leitungen stellen Rohre dar.

Zur Aerosolerzeugung können unterschiedliche Einrichtungen eingesetzt werden, solange diese die zugeleitete aktive Biomasse als feste oder flüssige Schwebteilchen, bzw. feste Schwebteilchen mit einem Fluidmantel aus dem jeweils eingesetzten Medium in den umzusetzenden reaktiven Eduktgasen herstellen können. In einer bevorzugten Ausführungsform wird eine Vorrichtung gemäß der vorliegenden Erfindung bereitgestellt wie hierin beschrieben, wobei die Einrichtung zur Aerosolerzeugung einen Ultraschallzerstäuber, einen Diffusor, oder eine Düse umfasst oder ist. Ist oder umfasst der Aerosolerzeuger eine Düse, so wird bevorzugt eine Piezodüse, eine Zweistoffdüse, eine Kegeldüse oder eine Zyklondüse eingesetzt.

Neben den dargelegten Bestandteilen kann die erfindungsgemäße Vorrichtung auch weitere enthalten, wobei die Zusammenstellung abhängig von den umzusetzenden Eduktgasen und/oder dem oder den herzustellenden Produkten ist. In einer bevorzugten Ausführungsform wird eine Vorrichtung wie hierin beschrieben bereitgestellt, wobei die Vorrichtung ferner eine oder mehrere Einrichtungen umfasst aus der Gruppe bestehend aus:

(a) Lichtquellen umfassend Tageslichtfenster, Leuchtstofflampen, Metalldampflampen, Natriumdampf-Niederdrucklampen, Natriumdampf-Hochdrucklampen und andere Licht emittierende Vorrichtungen wie LEDs oder OLEDs;
(b) Aufzuchtfermentern;
(c) Verdichtern;
(d) Nährlösungstauschern;
(e) Aerosolabscheidern;
(f) Auffangfermentern;
(g) Abscheidern;
(h) Pumpen;
(i) Leitungen welche die Einrichtungen gemäß den oben genannten Punkten (ii) bis (iv) sowie den Punkten (b) bis (h) miteinander und/oder mit dem Reaktor verbinden;
(j) Leitungen zur Zurückleitung der reaktiven Eduktgase in den Reaktor
(k) Leitungen zur Zurückleitung des wässrigen Mediums und/oder der aktiven Biomasse in den Reaktor oder den Aufzuchtfermenter;
(l) Leitungen zur Herausleitung der Produkte, aktiver Biomasse, inaktiver Biomasse, der Eduktgase und/oder des wässrigen Mediums aus der Vorrichtung;
(m) Kondensationsvorrichtungen; und
(n) Regeleinrichtungen umfassend Einrichtungen zur Erfassung und/oder Regelung von Temperatur, Druck, pH, Nährstoffen und Spurenelementen, Wassergehalt, Produktmenge, Durchflussmenge und/oder Aerosoltröpfchengröße;

Unter Aufzuchtfermentern werden hierbei allgemein Gefäße verstanden, welche zur Aufzucht, Vermehrung und/oder Aufbewahrung im lebensfähigen Zustand der erfindungsgemäß eingesetzten Mikroorganismen und/oder Zellen geeignet sind. Bevorzugt liegen die Mikroorganismen und/oder Zellen in einem Aufzuchtfermenter in einem zu den genannten Zwecken geeigneten Medium bzw. Fermentationsbrühe vor, welches beispielsweise notwendige Nährstoffe für die Mikroorganismen und/oder Zellen enthält.

Verdichter können in der erfindungsgemäßen Vorrichtung an unterschiedlichen Stellen eingesetzt werden, beispielsweise zur Verdichtung der Zellen und/oder Mikroorganismen auf dem Weg aus dem Aufzuchtfermenter zu dem Aerosolerzeuger und somit zum Reaktor. Durch eine Verdichtung kann hier eine höhere Partikeldichte in dem dann erzeugten Aerosol erzeugt werden und die bei der Verdichtung entzogene Nährlösung/Fermentationsbrühe wieder dem Fermenter zugeführt werden. Ein solcher Verdichter kann auch gleichzeitig auch als Nährlösungstauscher dienen, wobei die abgezogene Nährlösung aus der Vorrichtung geleitet und neue in den Aufzuchtfermenter eingebracht wird. Alternativ kann ein entsprechender Nährlösungstauscher auch als getrennte Einrichtung in der erfindungsgemäßen Vorrichtung enthalten sein. Zusätzlich oder alternativ kann ein weiterer Verdichter im Prozess hinter dem Aerosolreaktor angebracht werden, um die Abscheidung des Aerosols von der dann beladenen Biomasse zu erleichtern. Der Ausdruck beladen bezogen auf die Biomasse bezieht sich hier auf die Aufnahme der umzusetzenden Gase durch diese und gegebenenfalls auch schon auf die Beladung mit dem einen oder mehreren aus den Eduktgasen erzeugten Produkten.

Bevorzugt findet die Umsetzung der Eduktgase in dem Reaktor statt. Alternativ oder zusätzlich kann die Umsetzung oder ein Teil davon in nachgeschalteten Auffangfermentern stattfinden. Dies ist insbesondere der Fall, falls im Reaktor erst Zwischenprodukte hergestellt worden sind, die erst noch durch weitere Reaktionsschritte zu den Endprodukten umgesetzt werden müssen. Eine solche Umsetzung kann dann beispielsweise in solchen nachgeschalteten Auffangfermentern stattfinden.

Sowohl das eine oder mehrere hergestellte Produkte wie auch inaktive Biomasse können in der Vorrichtung aus dem Prozessstrom in geeigneten Abscheidern entfernt werden. Die Produktabscheider sind unterschiedlich gestaltet, je nach dem herzustellenden Produkt. Im einfachsten Fall erfolgt dies durch Diffusion aus dem jeweils eingesetzten Biomaterial und der gegebenenfalls vorhandenen Fluidhülle. Andere möglichen Wege sind im Abschnitt "Abtrennung von Produkten" dargelegt. Inaktive Biomasse, insbesondere inaktive Mikroorganismen und/oder Zellen verhalten sich beispielsweise physikalisch anders als aktive, indem sie in Lösung beispielsweise absinken. Hierdurch können sie in dem entsprechenden Abscheider, beispielsweise durch Sedimentation, Filtration und/oder Zentrifugen einfacher abgetrennt und aus der Vorrichtung, beispielsweise über eine Leitung entfernt werden.

Um den Fluss der Prozesssubstrate durch die Leitungen, wie z.B. von Biomasse und den Eduktgasen zu erleichtern, können auch Pumpen eingesetzt werden.

Ferner kann auch eine Vielzahl von Leitungen eingesetzt werden, die die einzelnen Komponenten der erfindungsgemäßen Vorrichtung miteinander verbinden. Insbesondere können auch Leitungen eingesetzt werden, die Prozesssubstrate wieder zurückleiten in den Prozessstrom. Ein Beispiel hierfür sind eine oder mehrere Leitungen, welche das Eduktgas nach dem Ausgang aus dem Reaktor wieder zurück in diesen bringen. Diese Leitungen können wieder zum Aerosolerzeuger führen, oder an anderen Stellen des Reaktors münden und dort zusätzliche Turbulenzen erzeugen, durch welche der Massetransfer der Eduktgase zu der Biomasse verbessert wird. Da die zurückgeführten Eduktgase niedriger konzentriert sind und möglicherweise auch schon Produkte enthalten, wird in einer Ausführungsform auf eine solche Rückleitung verzichtet. Stattdessen wird zur Erzeugung der erwähnten Turbulenzen das Eduktgas nicht nur durch den Aerosolerzeuger, sondern in einer Ausführungsform auch an mehreren weiteren Stellen des Reaktors in diesen eingeführt.

Kondensationsvorrichtungen und deren mögliche Ausgestaltungen sind schon weiter oben beschreiben worden. Ist eine Kondensationsvorrichtung als eine kalte Platte ausgeführt, so kann diese auch mit einem Temperatursensor und -regler ausgestattet werden, um die für die Kondensation notwendige Oberflächentemperatur zu erhalten. Ansonsten werden in einer bevorzugten Ausführungsform weitere Sensoren und Regler eingesetzt, beispielsweise um die Temperatur in den Fermentern und dem Reaktor zu erfassen und zu steuern. Für die effiziente Benutzung von Biokatalysatoren werden in entsprechenden Ausführungsformen sowohl in der erfindungsgemäßen Vorrichtung, wie in dem vorher beschriebenen erfindungsgemäßen Verfahren spezielle Temperaturen eingestellt, wobei diese Temperaturen bevorzugt in dem Bereich von etwa 20 bis 95°C liegen. Eine notwendige Temperaturerhöhung kann in besonderen Ausführungen auch durch Wärmequellen hervorgerufen werden, z. B. durch ablaufende exotherme Reaktionen oder durch elektrische Heizung oder durch Einstrahlung von elektromagnetischen Wellen oder Schall.

Das bevorzugte Verhältnis der Durchflussströme der Eduktgase zu der Biomasse ist weiter oben beschrieben worden. Die Dicke der Fluidschicht um die Biomasse, bzw. der Umfang der Aerosoltröpfchen kann bei der erfindungsgemäßen Vorrichtung durch entsprechende Gestaltung des Zerstäubers, beispielsweise durch Wahl der Düsenöffnung beeinflusst werden.

Dementsprechend betrifft die vorliegende Erfindung in einzelnen bevorzugten Ausführungsformen alternativ oder zusätzlich zu oben beschriebenen eine Verwendung der erfindungsgemäßen Vorrichtung zur Umsetzung von Synthesegas zu Ethanol und/oder Essigsäure und/oder Butanol und/oder Biomasse bzw. Bestandteilen davon; eine Verwendung zur oxidativen Umsetzung von Methan zu Methanol und/oder Biomasse bzw. Bestandteilen davon; eine Verwendung in der photokatalytischen Umsetzung von $CO_2$ zu $H_2$ und/oder Biomasse bzw. Bestandteilen davon; eine Verwendung zur Umsetzung von Synthesegas zu Methan; und eine Verwendung der erfindungsgemäßen Vorrichtung zur Umwandlung und/oder Reinigung von Gasen/Flüssigkeiten, z.B. von Stickoxiden.

Abgrenzung zu bestehenden Verfahren und Erfindungen

Nach Kenntnis der Erfinder wird die Aerosoltechnik in der mikrobiologischen Produktion bisher nicht angewendet. Während die Verwendung von Aerosolen in der Verbindung mit Mikrobiologie zwar für den Abbau nicht gasförmiger Schadstoffe, oder auch die Untersuchung medizinisch oder umwelttechnisch relevanter Faktoren genutzt wird, wurde diese Technik bisher nicht auf die Umsetzung gasförmiger Stoffe oder für die biotechnologische Produktion verwendet. Chemische Produktionsverfahren dagegen, in denen Aerosoltechniken angewendet werden, existieren zwar, unterscheiden sich aber von der beschriebenen Erfindung dadurch, dass keine Biokatalysatoren involviert sind.

Figuren und Ausführungsbeispiele

Der Erfindung ist nachstehend anhand von Ausführungsbeispielen und Figuren näher erläutert, ohne dass dies als auf eine bestimmte Ausführungsform beschränkend verstanden werden soll.

Es zeigen:

Figur 1: Aerosolfermenter Verfahrensprinzip/Skizze: (A) ein Schema der erfindungsgemäßen Vorrichtung, (B) eine Zelle, beispielsweise eines Mikroorganismus umgeben von wässriger Lösung in die das Gas einfach eindringen und auf kurzem Weg zur Zelle durchdringen kann.

Figur 2: eine Ausführungsform der erfindungsgemäßen Vorrichtung mit weiteren Einrichtungen, wie sie beispielsweise bei Umsetzung von Synthesegas aus Pyrolyse von Holzpellets zur Erzeugung von Ethanol und/oder Essigsäure eingesetzt werden kann.

In Figur 1 wird ein Schema der erfindungsgemäßen Vorrichtung und eine in einem Aerosoltropfen eingeschlossene Beispielzelle gezeigt. Die Fermentationsbrühe (5) wird mit den Mikroorganismen und eventuellen Zuschlagstoffen in den Gärraum (6) eingesprüht. Hierzu können verschiedene Vorrichtungen, wie Kegeldüsen, Zweistoffdüsen oder ähnliche verwendet werden. In den entstehenden Tröpfchen sind die aktiven mikrobiologischen Systeme enthalten und nur von einem geringen Flüssigkeitsfilm umgeben.

Der Massetransfer der Eduktgase zu dem aktiven Zellteilchen ist durch große Gas/Flüssigkeitsoberfläche und erhöhte Diffusionskoeffizienten und Trefferwahrscheinlichkeiten stark erhöht im Vergleich zu üblichen Methoden, wie zuvor beschrieben.

Figur 2 zeigt eine weitere beispielhafte Komponentenzusammenstellung der Vorrichtung der vorliegenden Erfindung, wie sie beispielsweise zur Umsetzung von Synthesegas (2), entstehend z.B. in einem Pyrolyseofen (17) zu Ethanol oder Essigsäure eingesetzt werden kann. Die aktive Biomasse (4a) wird in einem Aufzuchtfermenter (5a) aufgezogen und durch einen Zellverdichter (19), aus welchem ein Teil der Fermentationsbrühe abgezogen und erneuert zum Aufzuchtfermenter (5a) rezirkuliert wird (18), zu dem Aerosolerzeuger (20) geleitet. Das Gas (2) zusammen mit der aktiven, unbeladenen Biomasse (4a) werden durch den Aerosolerzeuger (20) in Aerosol umgesetzt und als solches in den Gärraum mit Eduktgas (6) in den Reaktor (6a) eingebracht. Die dann beladene Biomasse wird durch den Verdichter (13) in den Aerosolabscheider (14) geleitet, aus dem zum Teil das noch vorhandene Eduktgas zum Reaktor (6a) zurückgeführt werden kann (16). Die beladene Biomasse (21) wird weiter zu einem Auffangfermenter (15) durchgeleitet, in dem gegebenenfalls weitere Reaktionen stattfinden können. Ist dies nicht notwendig kann die beladene Biomasse (21) auch direkt zum Abscheider für Produkt(e) (12) geleitet werden, aus welchem das Produkt(e) herausgetrennt und aus der Vorrichtung abgeleitet werden können (11). Die Biomasse wird weitergeleitet zum entsprechenden Abscheider (9), der inaktive Biomasse abscheidet und aus der Vorrichtung leitet (10), während noch aktive, nun unbeladene Biomasse ins Verfahren rückgeführt wird (8).

Ausführungsbeispiele

(Die Bezugszeichen beziehen sich auf Figur 1 und 2)

Anwendungsbeispiele für den Einsatz des beschriebenen Verfahrens sind unter anderem die mikrobiologische Umsetzung von Synthesegas oder dessen Bestandteilen zu Kohlenwasserstoffen wie zum Beispiel Ethanol oder Butanol. Die Herstellung von Synthesegas ist beispielsweise in:: Gas Fermentation for Commercial Biofuels Production, Fung Min Liew, Michael Köpke and Sean Dennis Simpson, DOI : 10.5772/52164 beschrieben. Es kann jedoch auch jedes andere Synthesegas erfindungsgemäß verwendet werden. Eine weitere Anwendung des erfindungsgemäßen Verfahrens ist z.B. die mikrobielle Umsetzung einer Mischung aus $CO_2$ und $H_2$ zu Methan.

**Beispiel 1**

Ein gemäß dieser Vorschrift erhaltenes Synthesegas, das bei einer thermischen Behandlung (Vergasung) organischer Reststoffe in Sauerstoffatmosphäre bei 480-800°C erhalten wird, wird fermentativ umgesetzt. Die Hauptbestandteile des Synthesegases aus der oben erwähnten thermischen Behandlung sind neben $N_2$ die reaktiven Bestandteile von üblichem Synthesegas CO, $CO_2$ und $H_2$. $CH_4$ ist nur zu einem geringen Anteil enthalten.

Weitere Bestandteile wie Asche, Teere, Stickoxide NOx, und Schwefelverbindungen, die oft in Synthesegasen enthalten sind, sind aufgrund des angewandten Verfahrens nicht, oder nur in sehr geringen Mengen vorhanden. Aus der thermischen Behandlung (Pyrolyse) von 250 kg organischen Reststoffen (Holzpellets) wurden in dem verwendeten Verfahren Substratgasmengen von 13,5 kg $H_2$, 192,8 kg CO und 87,2 kg $CO_2$ erhalten.

Tabelle 1: Zusammensetzung des Synthesegases, gewonnen aus thermischer Behandlung organischer Reststoffe, das als Eduktgas in die Bioaerosolfermentation zur Erzeugung von Ethanol eingesetzt wird

| | Materie | Massen-bilanz [kg/h] | Volumenbilanz [Nm3/h] | Anteil | Stoffmengenbilanz n [mol/h] |
|---|---|---|---|---|---|
| Einsatz - thermisches Verfahren | | | | | |
| | organischer Reststoff | 250 | 1,25 | | |
| | O2 | 85,66 | 60 | | 2676,9 |
| Ergebnis - thermisches Verfahren== Einsatz - Fermentation | | | | | |
| | N2 | 5,09 | 4,07 | 0,01 | 181,6 |
| | H2O | 8,62 | 10,73 | 0,03 | 478,7 |
| | CH4 | 4,50 | 6,29 | 0,02 | 280,6 |
| | H2 | 13,51 | 150,22 | 0,41 | 6702,0 |
| | CO | 192,81 | 154,29 | 0,42 | 6883,6 |
| | CO2 | 87,18 | 44,4 | 0,12 | 1980,9 |
| | Gesamt | 311,71 | 370 | 1,00 | 16507,5 |

Die Umsetzung des Synthesegases zu Ethanol ist abhängig von der Menge und Zusammensetzung der reaktiven Bestandteile CO, $CO_2$ und $H_2$ des Substratgases. Die Stöchiometrie der Umsetzung der Substrate zu Ethanol erfolgt nach Gleichungen IA und IB.

IA $6\,CO + 3\,H_2O \rightarrow C_2H_5OH + 4\,CO_2$ $\Delta G° = -217.9$ kJ/mol

IB $2\,CO_2 + 6\,H_2 \rightarrow C_2H_5OH + 3\,H_2O$ $\Delta G° = -97.3$ kJ/mol

Die maximale Ausbeute auf Grundlage der in Tabelle 1 dargestellten Angaben der Substratmengen ist in Tabelle 2 zusammengefasst. Bei optimaler Umsetzung des Synthesegases entspricht der maximale Ethanol-Ertrag ~104 kg pro 250 kg organischem Reststoff (Holzpellets). Die vollständige Umsetzung des erhaltenen $CO_2$ ist dabei aufgrund der geringen $H_2$ Menge selten gegeben. Unter zusätzlicher Bereitstellung von ~25 kg $H_2$ kann gemäß Gleichung IB die vollständige Umsetzung des Substratgases erzielt werden. Der Ethanol-Ertrag wird dadurch auf ~190 kg pro 250 kg organischer Reststoff nahezu verdoppelt.

Tabelle 2: Substratmengen in den Produkten

| Ergebnis - thermische Reststoffbehandlung & Fermentation | | | | | |
|---|---|---|---|---|---|
| | Materie | Massen-bilanz [kg/h] | | | Stoffmengenbilanz n [mol/h] |
| | H2 | 0 | | | 0 |
| | CO | 0 | | | 0 |
| | CO2 | 190,82 | | | 4335,97 |
| | C2H5OH | 104,3 | | | 2264,28 |

Um die optimalsten Bedingungen der Zusammensetzung des Synthesegases für einen effizienten erfindungsgemäßen Prozess zu finden, wurden Synthesegase unterschiedlicher Gaszuströme eingesetzt. Verglichen wurden dabei die Ergebnisse der thermischen Behandlung von 250 kg/h organischem Reststoff (Holzpellets) unter Zustrom von reinem Sauerstoff, reiner Luft und mit einem Gemisch aus Luft und Wasserdampf (25%). In Tabelle 3 sind die Zusammensetzungen der thermischen Behandlung, sowie die Ergebnisse unter (optimaler) Fermentation (d. h. vollständiger Umsatz der Substratgase, Umsatz nur zu Ethanol) zusammengefasst.

Für die fermentative Umsetzung sind weder $N_2$ noch $CH_4$ von Bedeutung. Lediglich die genannten weiteren Bestandteile wie Asche, Teere, Stickoxide NOx, und Schwefelverbindungen stellen ein Risiko in der fermentativen Verarbeitung dar, können jedoch durch gängige Reinigungsmethoden soweit entfernt werden, dass eine Prozessstörung ausgeschlossen werden kann. Eine Verunreinigung durch $O_2$ muss auf jeden Fall vermieden werden, da nach Forschungsergebnissen Sauerstoff aufgrund seiner oxidativen Wirkung den Prozess stark stört und bei Konzentrationen über 5% auf jeden Fall toxische Eigenschaften aufweist.

Tabelle 3: Erwarteten Zusammensetzungen der thermischen Behandlung organischer Reststoffe und Ergebnisse bei optimaler Fermentation.

| Zusammensetzung des Synthesegases unter unterschiedlichen Bedingungen | | | |
| --- | --- | --- | --- |
| -Medium der thermischen Behandlung | O2 | Luft | Luft + Wasserdampf (25%) |
| N2 | 1,1% | 40,5% | 36,2% |
| H2O | 2,9% | 2,9% | 2,9% |
| CH4 | 1,7% | 1,0% | 2,8% |
| H2 | 40,6% | 23,9% | 28,3% |
| CO | 41,7% | 22,3% | 13,4% |
| CO2 | 12,0% | 9,4% | 16,4% |
| Gesamt | 100,0% | 100,0% | 100,0% |
| Gesamtvol [Nm/h]/250kgor ganischer Reststoff | 370 | 620 | 620 |
| Ergebnisse der Fermentation unter optimalen Bedingungen | | | |
| *[kg/h]* | | | |
| *CO* | 0,0 | 0,0 | 0,0 |
| *H2* | 0,0 | 0,0 | 0,0 |
| *CO2* | 190,8 | 198,4 | 193,6 |
| *EtOH* | 104,3 | 98,1 | 88,6 |
| *HAc* | 0,0 | 0,0 | 0,0 |

Die fermentative Umsetzung von Synthesegas (einer Mischung aus $H_2$, CO und $CO_2$) beruht auf der Fähigkeit autotropher acetogener Bakterien unter strikt anaeroben Bedingungen ihren Energiebedarf durch den reduktiven Abbau von Kohlenstoffoxiden zu decken. Grundlage für diese Fähigkeit bildet ein nichtzyklischer Stoffwechselweg zur Kohlenstoffassimilation, der als Acetyl-CoA- oder Wood-Ljungdahl-Weg bezeichnet wird. Unter Verbrauch von Wasserstoff und Kohlenstoffoxiden erfolgt durch biokatalytische, enzymatische Umsetzung ein Aufbau von $C_2$-Kohlenwasserstoffen, deren gemeinsame Zwischenstufe das Coenzym Acetyl-CoA bildet. Besondere Merkmale des Wood-Ljungdahl-Weges unicarbonotropher Bakterien ist dabei die Koexistenz zweier Zweige (Methyl- und Carbonyl-Zweig), die neben einer Umsetzung von $CO_2$ und $H_2$ auch die Umsetzung von CO ermöglichen. Wesentlich für die fermentative Produktion von Ethanol ist die Existenz zweier Abbauwege der Zwischenstufe Acetyl-CoA im Folgeschritt: Unter Einfluss mikrobieller Regelfaktoren erfolgt eine Umsetzung entweder in Ethanol (EtOH) oder in das Konkurrenzprodukt Essigsäure (HAc).

Zusammenfassend kann der mikrobakterielle Umsatz von Synthesegas durch die folgenden biochemischen Gleichungen beschrieben werden:

IA $6\ CO + 3\ H_2O \rightarrow C_2H_5OH + 4\ CO_2\ \Delta G° = -217.9$ kJ/mol

IB $2\ CO_2 + 6\ H_2 \rightarrow C_2H_5OH + 3\ H_2O\ \Delta G° = -97.3$ kJ/mol

IIA $4\ CO + 2\ H_2O \rightarrow CH_3COOH + 2\ CO_2\ \Delta G° = -154.9$ kJ/mol

IIB $2\ CO_2 + 4\ H_2 \rightarrow CH_3COOH + 2\ H_2O\ \Delta G° = -75.3$ kJ/mol

Dabei sind durch A und B die Abbauwege über den Methyl- und den Carbonyl-Zweig bezeichnet. Der gewünschte Abbau zu Ethanol wird durch Gleichungen IA und IB bezeichnet, während der Abbau zu dem Konkurrenzprodukt HAc durch Gleichungen IIA und IIB beschrieben wird.

Unter die erwähnten mikrobiellen Regelfaktoren, die der Steuerung und Kontrolle des fermentativen Abbaus dienen, fallen äußere Einflüsse wie Druck, Temperatur, pH, Leitfähigkeit, Redox-Potential sowie Hemmstoffe und verfügbare Nährstoffe, aber auch mikrokulturelle Faktoren wie Zusammensetzung der eingesetzten Kulturen, Lebenszyklus und Populationsdichte. Zusammen bilden sie ein komplexes Gefüge das wesentlich für den Verlauf des fermentativen Prozesses ist und somit die Effizienz einer Fermentationsanlage bestimmt.

Die Regelung der Umsatzeffizienz und der Produktverteilung durch mikrobakterielle und verfahrenstechnische Regelfaktoren findet in den unterschiedlichen Prozessschritten der Fermentation ihren Einsatz. Regelfaktoren können dabei in einzelnen Prozessschritten konträre Auswirkungen haben. Die Aufschlüsselung und Analyse des Fermentationsprozesses in die einzelnen Teilschritte ist daher wesentlich für den gezielten Einsatz der Regelfaktoren.

Die Zerlegung der fermentativen Umsetzung von Synthesegas zu Ethanol in die wesentlichen Teilschritte und die Auswirkungen ihrer Regelparameter wird im Folgenden zusammengefasst.

1. Übergang der Substrate von der gasförmigen Phase in die flüssige Phase

Die Effizienz des Überganges der Substrate in die flüssige Lösung wird durch Massetransportkoeffizienten beschrieben. Aufgrund der geringen Löslichkeit der SubstratGase in Wasser gilt dieser Schritt als ein wesentlicher beschränkender Faktor für die effiziente Fermentation von Synthesegas. Temperatur, Druck, Oberflächengröße sowie Art der Oberfläche sind wesentliche Regelfaktoren des zugrundeliegenden Massetransportes. Dieser Übergang wird durch die erfindungsgemäß eingesetzte Aerosolbildung und gegebenenfalls später auftretende/erzeugte turbulente Strömungen begünstigt.

2. Transport der Substrate von der Gas-Flüssigkeitsgrenzfläche zu der Zellwand

Der Substrattransport in der Flüssigkeit wird durch Diffusion beschrieben. Einflussfaktoren sind unter anderem die Weglänge zwischen Gas-Flüssigkeitsgrenzfläche und der Zelle, sowie die Viskosität der Flüssigkeit, die im Falle von Biofilmen von wässrigen Eigenschaften abweichen kann. Diesem Teilschritt wird im Allgemeinen geringere Bedeutung zugeordnet, er wird allerdings ebenfalls in den erzeugten, feinen Aerosoltröpfchen durch die geringe Fluidschichtdicke begünstigt.

3. Eintritt der Substrate ins Zellinnere

Im Falle kleiner Moleküle wie der verwendeten Substratgase wird die Aufnahme in das Zellinnere durch Diffusion beschrieben und hängt im Wesentlichen von der in Teilschritt 1. erzeugten Konzentration der Substratgase in Lösung ab.

4. Umwandlung der Substrate in CoAc-A

Die Umwandlung der Substrate in die Zwischenstufe CoAc-A wird wesentlich durch die Zusammensetzung der Nährlösung, ihr pH-Wert und ihr Redoxpotential bestimmt. Hemmstoffe können als Inhibitoren die Umsetzung stören, während sich andererseits die Gegenwart reduktiver Reagenzien positiv auf die Umsetzung auswirken kann.

5. Umwandlung von CoAc-A in die Produkte

Die Umwandlung der Zwischenstufe in die Produkte (EtOH oder HAc) ist eine der wichtigsten Teilschritte der fermentativen Ethanol Produktion. Er hängt wesentlich von dem mikrobiellen Lebenszyklus ab und wird durch Umgebungsvariablen, wie auch durch biologische Substanzen mit Signalwirkung gesteuert.

6. Austritt der Produkte aus der Zelle

Ähnlich wie der Substrattransport erfolgt auch der Produkttransport durch die Zellwand durch Diffusion und wird wesentlich durch die Produktkonzentration in der externen Lösung bestimmt. Findet der Transport aus der Zelle nicht statt, so wirkt sich die erhöhte Produktkonzentration im Zellinneren negativ auf die Produktion aus. Für einen effizienten Umsatz ist daher eine möglichst stetige und effiziente Abtrennung der Produkte bereits im laufenden Prozess erwünscht.

7. Trennung und Aufreinigung der Produkte

Neben den in Teilschritt 6. erwähnten Auswirkungen auf die mikrobiologische Umsatzeffizienz hat dieser Teilschritt eine wesentliche Bedeutung für die wirtschaftliche Anwendung. Der Energieaufwand für die Aufreinigung wässriger Ethanollösungen ist sehr hoch und steigt mit sinkender Ethanolkonzentration. Eine wesentliche Anforderung im Zuge der industriellen Skalierung von Fermentationsanlagen ist daher die Integration eines effizienten Trennverfahrens in den laufenden Prozess.

Für die Gewährleistung eines effizienten, langlebigen und kontinuierlichen Verlaufes des Fermentationsprozesses müssen weiterhin folgende mikrobielle Faktoren berücksichtigt werden:

1. Aufbau der Biomasse

Bei bekannten Fermentationsanlagen wird der maximal mögliche Umsatz von Synthesegas durch die Anzahl der Zellen bestimmt, während bei gleicher Zellzahl die Anlagengröße durch die erreichte Zelldichte bestimmt wird. Das exponentielle Wachstum der Biologie, das heißt der Aufbau von Biomasse, in dem Zellzahl und -dichte bestimmt werden erfolgt unter anderen mikrobiologischen Bedingungen als das Leben der Bakterien unter konstanter Zellzahl. Des Weiteren wirkt sich wie oben erwähnt die Wachstumsphase auf die Produktverteilung aus. Die Anfahrphase und der kontinuierliche Betrieb der Fermentationsanlage muss daher optimal auf die entsprechenden Lebenszyklen abgestimmt werden. Diese Faktoren fallen in dem erfindungsgemäß eingesetzten Verfahren weitestgehend weg. Es besteht kein Reaktionstank mehr mit einer limitierenden Zelldichte. Vielmehr können immer wieder neue Zellen durch und mit dem Eduktgas durch den Reaktor (6, 6a) durchgeschleust werden. Durch die reduzierte flüssige Phase um die Zellen wird die Austauschoberfläche vergrößert, sodass der Massentransfer effizienter stattfinden kann, als in den bekannten Reaktoren, in denen die Zellen gelöst im Mediumbad vorliegen. Durch die dünne Fluidschicht um die Zellen kann ferner deren Dichte im Reaktor gegenüber den üblichen Flüssigreaktoren erhöht werden. Die gleichmäßige Menge und Dichte der Zellen im Aerosolstrom kann erreicht werden, wie im nächsten Punkt beschrieben.

2. Erhalt der Dichte aktiver Biomasse

Der kontinuierliche Betrieb der Fermentationsanlagen nach Stand der Technik erfordert den Erhalt der Zelldichte. Die ausreichende Versorgung durch Nährstoffe, wie die Vermeidung ungewünschter Hemmstoffe muss daher gewährleistet sein. Um rechtzeitig geeignete Maßnahmen gegen Störungen ergreifen zu können werden üblicherweise Kontrollmaßnahmen eingeführt. Diese Maßnahmen werden hier auch in Bezug auf den Aufzuchtfermenter (5, 5a) getroffen. Sie sind jedoch nicht Wesentlich in Bezug auf den Gastransfer, da weitestgehend konstante Zellmengen und Dichten im Aerosolstrom erreicht werden können, indem die Zellmenge und Dichte in dem Verdichter (19) nach dem Aufzuchtfermenter (5, 5a) angepasst wird.

3. Abtrennung verbrauchter Biomasse

Bakterien haben eine kurze Lebensdauer. Abgestorbene Biomasse kann Hemmungen auslösen und zu verfahrenstechnischen Schwierigkeiten führen. Des Weiteren können sich Fremdkulturen entwickeln, die sich negativ auf die bestehende Kultur auswirken. Die Abtrennung verbrauchter Biomasse stellt daher einen ebenfalls wichtigen Aspekt in der Anlagenentwicklung dar und kann in Abhängigkeit des Designs des Bioreaktors unterschiedlich aufwendig sein. Sie wird gemäß der Erfindung im entsprechenden Abscheider (9) durchgeführt.

Wichtig für die Effizienz des Fermentationsprozesses ist außerdem die Geschwindigkeit, in der die Umsetzung der Substratgase zu Ethanol erfolgt. Für eine vollständige Umsetzung der Substratgase muss in den bekannten Fermentern die Umsatzgeschwindigkeit dem vorhergehenden thermischen Prozess zur Erzeugung der Eduktgase möglichst angeglichen werden. In dem erfindungsgemäßen Reaktor (6, 6a) kann diese Limitierung ebenfalls dadurch umgangen werden, dass die Biomasse sich nur kurzfristig im Reaktor aufhält und ständig durch Nachschub von neuer, unbeladener Biomasse erneuert wird. Durch Parallelschaltung mehrerer Reaktoren, selektive Steuerung dieser Reaktoren, schnelles Abscheiden der Produkte und Recycling der Biomasse kann hier der Umsatz des Synthesegases reguliert und beschleunigt werden.

Für eine effiziente Umsetzung von Synthesegas zu Ethanol gemäß der vorliegenden Erfindung lassen sich zusammenfassend die folgenden Anforderungen an die Schlüsselparameter stellen:

1. Hohe Zelldichte

2. Hoher Substrat-Massetransfer von der Gasphase in die Reaktionslösung

3. Hohe Umsatzgeschwindigkeit

4. Effektive Produkt-Separation aus der Reaktionslösung

5. Geringe Produkt-Konzentration der separierten Lösung

Wobei die erfindungsgemäßen Verfahren und Vorrichtungen Verbesserungen in Bezug auf alle der Parameter bereitstellen, wie oben dargelegt wurde.

Verfahrensablauf :

Das erzeugte Synthesegas wird mit den Bakterien in den Reaktor (6, 6a) eingeleitet, wo die Umsetzung der reaktiven Bestandteile durch die Mikroben zu Ethanol erfolgt. Die Bakterien (3) befinden sich in einer dünnen Schicht aus wässrigem Medium (1), dessen Nährgehalt nach Rezirkulation in den Aufzuchtfermenter (5, 5a) bzw. im Auffangfermenter (15) durch die Nährlösung ausgeglichen wird. Der Aufzuchtfermenter (5, 5a) weist typischerweise eine Zufuhrmöglichkeit für Nährstoff/Additiv auf. Die Abtrennung verbrauchter Biomasse (9) sowie der erzeugten Produkte Ethanol und Essigsäure erfolgt im Laufe des Prozesses. Die Abtrennung erfolgt separat in einem externen Kreislauf (9, 12) außerhalb des Reaktors (6, 6a). Die separierte verbrauchte Biomasse wird in einem Behälter gesammelt (9) und unverbrauchte Nährstoffe werden dem Aufzuchtfermenter (5, 5a) wieder zugeführt. Das abgetrennte wässrige Ethanol wird anschließend bis zum gewünschten Grad aufgereinigt.

Wesentlich für die Produktausbeute bzw. ökonomisch vorteilhafte Produktion ist in dem beschriebenen Verfahren der Aufbau der erfindungsgemäßen Vorrichtung. Im Gegensatz zu der Mikrobiologie in Lösung sind hier die Zellen nur von einem dünnen Flüssigkeitsfilm (1) überzogen. Vorteile sind kurze Diffusionsgeschwindigkeiten in der wässrigen Phase, wodurch die erforderliche Gaskonzentration in Lösung und somit der Zielmassetransfer der Gassubstrate herabgesenkt wird. Im Gegensatz zu immobilisierter Mikrobiologie, bei der auftretende Faulprozesse und die erschwerte Abtrennung verbrauchter Biomasse einen großen Nachteil darstellen, die die technische Handhabung, insbesondere mit steigender Oberfläche erschweren, ist erfindungsgemäß hier die Vermeidung von Faulprozessen durch erleichterte Abtrennung inaktiver Biomasse gegeben.

Zusammengefasst basiert die hier beispielhaft beschriebene Ausführungsform des erfindungsgemäßen Verfahrens auf der räumlichen und zeitlichen Trennung der unterschiedlichen Prozessschritte:

1. Zellaufzucht

2. Zellverdichtung

3. Substrataufnahme und -umwandlung durch Mikrobiologie

4. Separation von Produkten, der Nährlösung, sowie aktiver und verbrauchter Biomasse

5. Produktaufbereitung

6. Restgasrückführung/-verbrauch

a. Separation von Ethanol

i. Membrantrennverfahren

ii. Niederdruck Membranverfahren

Vorteil dieser Trennung von Prozessschritten ist die Möglichkeit, die Prozessvariablen an die unterschiedlichen Anforderungen der Prozessschritte anzupassen, um die optimale Effizienz des Verfahrens zu gewährleisten. Die Neuheit des entwickelten Verfahrens liegt dabei in dem Prozessschritt der Substrataufnahme im Aerosol, der nach allgemeinem Kenntnisstand eine der wesentlichen Herausforderungen für die Wirtschaftlichkeit der fermentativen Ethanolgewinnung darstellt. Im Gegensatz zu üblichen Verfahren erfolgt hier durch Generation eines Bioaerosols die Substrataufnahme in Form von freien Mikroben in der Gasphase. Vorteile dieses Prozesses sind die energiearme Maximierung der Grenzflächen Gas-Flüssigkeit und Flüssigkeit-Zellwand mit einhergehender Maximierung des Gas-Zellverhältnisses unter gleichzeitiger Reduktion der Weglängen in Lösung und einfacher Handhabung und Wiederaufbereitung.

Eine Skizze der beispielhaften Ausführungsform des erfindungsgemäßen Verfahrens ist in Fig. 2 dargestellt.

Im Folgenden werden die einzelnen Prozessschritte kurz dargestellt.

1. Zellaufzucht

a. Die Zellaufzucht der freischwebenden Biologie findet in einem ersten Fermentationsbehälter (5, 5a) statt. Optimale Wachstumsbedingungen werden durch geeignete Zusammenstellung der Nährlösung und Umgebungsvariablen wie Temperatur und Druck eingestellt. Als Aufzuchtbehälter (5, 5a) kann ein handelsüblicher kontinuierlicher Reaktor mit entsprechenden Vorrichtungen für optimalen Betrieb, auf die hier nicht näher eingegangen werden soll, verwendet werden. Eine energetisch effiziente Durchmischung der Fermentationsbrut im Reaktor kann durch Gaseinspeisung des Synthesegases mit geeigneten Vorrichtungen (Microbubble-Disperser) erreicht werden. Unverbrauchtes Synthesegas wird anschließend dem Prozess wieder zugeführt. Unter optimalen Bedingungen kann während der Aufzucht eine Zelldichte von näherungsweise $10^6$/ml bis $10^{11}$/ml typischerweise ca.$10^8$/ml in erreicht werden.

2. Zellverdichtung

a. Die weitere Erhöhung der Zelldichte, sowie die erforderliche Separation der Biologie von produktionshemmenden Stoffen werden in Anschluss an die Aufzucht erreicht. Für diesen Schritt stehen als Einrichtungen Zentrifugen oder Hydrozyklone zur Auswahl. Die abgetrennte Nährlösung wird in den Aufzuchtfermenter (5, 5a) zurückgeführt (18). Die Lösung mit der konzentrierten aktiven Biologie (4, 4a) wird zu dem Produktionsreaktor (6, 6a) geführt.

3. Substrataufnahme durch Aerosolbildung

Mit Hilfe von Düsen als Aerosolerzeuger (20) wird die Fermentationsbrühe (4, 4a) in den Gasstrom (2) der Substratgase eingebracht. Die Struktur des erzeugten Aerosols besteht aus Agglomeraten von aktiven Bakterien umgeben von einem Flüssigkeitsfilm. Sowohl die Größe der Agglomerate, wie auch die Schichtdicke des Flüssigkeitsfilmes hängen von der Wahl der Düsen ab. Ziel ist dabei gleichzeitig sowohl die Partikelgrößen (Radien $\leq 3\mu m$), wie auch die Schichtdicke des Flüssigkeitsfilmes (zwischen $0,5\mu m$ bis $200\mu m$, bevorzugt soweit möglich $\sim 0\mu m$) zu minimieren ohne die Zellen durch Austrocknung zu inaktivieren. Das so erzeugte Aerosol zeichnet sich durch eine hohe Gas/Zell- bzw. Gas/Flüssigkeits-Oberfläche aus. Nach ersten Ergebnissen kann in diesem Verfahren bei Einsatz eines Synthesegases aus der thermischen Behandlung organischer Reststoffe mit Luft eine Verbesserung des Zell-Gas Verhältnisses um den Faktor 10 im Vergleich zu Flüssigkeitsreaktoren unter Normaldruck erreicht werden. Da auftreffende Gasmoleküle direkt in die Zellwand aufgenommen werden, ist die Gaskonzentration in den Agglomeraten lediglich durch das Diffusionsverhalten der Gase in das Zellinnere und die Geschwindigkeit der anschließenden mikrobiologischen Umsetzung bestimmt. Die biologische Umsetzung sorgt dabei für einen stetigen Konzentrationsabbau im Zellinneren, wodurch das Diffusionsgleichgewicht positiv beeinflusst wird. Wesentlich für die Effizienz des Verfahrens ist die Kollision von Substratgasen mit Zellagglomeraten. Diese ist gekennzeichnet durch die Aerosoloberfläche, die Aerosollebenszeit, die relative Geschwindigkeit zwischen Aerosolpartikeln und Eduktgasmolekülen wie auch die Gasdichte. Während durch Wirbelströme bei einem durchschnittlichen Radius der Agglomerate von $3\mu m$ eine Lebenszeit von bis zu 1.5 Stunden erreicht werden kann, kann alternativ durch Kompression die Gasdichte erhöht werden.

4. Mikrobiologische Umwandlung

Je nach Geschwindigkeit der mikrobiellen Umsetzung findet diese bereits im Aerosolreaktor oder erst in einem anschließenden Auffangreaktor statt. Aufgrund der kurzen Diffusionszeiten der Substratgase durch die Zellwand (Mikrosekundenbereich) findet die Reaktion bei ausreichender Verweilzeit im Reaktor zum Teil oder bereits vollständig im Aerosolreaktor statt. Durch stetige Zufuhr von Eduktgasen, bzw. Eduktgas-Aerosol-Gemisch sollte die durch die Reaktion stattfindende Reduktion des Gasvolumens ausgeglichen werden und ein leichter Überdruck in dem Reaktor erhalten werden.

5. Separation

Abtrennung findet bereits im Aerosolreaktor statt (Molekularsiebe, Kondensation, Membrantrennverfahren). Zum Teil oder alternativ werden auch übliche Verfahren für die Separation außerhalb des Reaktors genutzt.
Für den erneuten Einsatz der aktiven Biologie (8) wird im Anschluss an die Verweilzeit in dem Anschlussreaktor die inaktive Biomasse durch übliche Verfahren (Sedimentation) abgetrennt (9).

Betriebsparameter

Zur Gasumsetzung werden in der beispielhaft beschriebenen Ausführungsform mesophile Mikroben der Art *Clostridium ljungdahlii* verwendet. Eine beispielhafte Ausgangszusammensetzung der Nährlösung für die Aufzucht ist in Tabelle 4 zusammengefasst. Diese kann bei der weiteren Aufzucht beibehalten oder modifiziert werden.

Tabelle 4: Ausgangszusammensetzung der Nährlösung für die Aufzucht von Clostridien.

| Substanz | Menge | Substanz | Menge |
|---|---|---|---|
| *Spurenelement-Lösung* | [g/l] | *Aufzucht Nährlösung* | [g/l] |
| EDTA | 10 | K2HPO4 | 0,57 |
| NiCl2 x 6H2O | 0,32 | KH2PO4 | 0,75 |
| MnCl2 x 4 H2O | 0,32 | MgSO4 x 7 H2O | 0,71 |
| FeCl2 x 4 H2O | 3,68 | MnSO4 x H2O | 0,01 |
| CoCl2 x 6 H2O | 0,4 | FeSO4 x 7 H2O | 0,01 |
| ZnCl2 | 0,1 | NaCl | 1 |
| CuCl2 | 0,03 | Hefeextrakt | 5 |
| KAl(SO4)2 x 12 H2O | 0,32 | Cystein | 1 |
| H3BO3 | 0,6 | Asparagin | 2 |
| NaOH | 0,4 | Glucose | 50 |
| Na2MoO4 x 2 H2O | 0,1 | Spurenelement-Lösung | 1% |
| Na2SiO3 x 5 H2O | 0,1 | Vitamin-Lösung | 1% |
| Na2SeO3 x 5 H2O | 0,1 | | |
| Na2WO4 x 2 H2O | 0,1 | | |
| *Vitamin-Lösung* | [mg/l] | | |
| Biotin | 20 | | |
| Folsäure | 50 | | |
| Pyridoxin | 500 | | |
| Thiamin (Vitamin B1) | 200 | | |
| Riboflavin | 100 | | |
| Nikotinamid | 200 | | |
| Panthothensäure | 100 | | |
| Vitamin B12 | 100 | | |
| p-Aminobenzoesäure | 100 | | |
| Liponsäure | 50 | | |

Die optimale Betriebstemperatur für die Fermentation mit diesen Mikroben liegt bei etwa 41 °C. Bei Einsatz anderer Bakterien, z.B. thermophiler Stämme werden höhere Temperaturen eingesetzt. Gasdrücke in der Anlage befinden sich zunächst kurz über Atmosphärendruck, bei mindestens 1100 mbar, bevorzugt bis 2000 mbar.

Für die Dimensionierung des Reaktors werden Parameter wie benötigte Gasmengen für einen kontinuierlichen Betrieb der Anlage, sowie die Anpassung der Gasgeschwindigkeiten im Reaktor durch Wahl des Reaktorradius und die Steuerung der Verweilzeit durch die Reaktorlänge unter gleichzeitigem Aufrechterhalten des Aerosols, sowie die Geschwindigkeit des erzielten Massentransfers in Abhängigkeit von Größe und Zusammensetzung der erzeugten Aerosolpartikel, respektive der Reaktionsgeschwindigkeit berücksichtigt. Für eine optimale Substrataufnahme ist eine vollständige radiale Durchmischung im Aerosolfermenter optimal. Um dies zu erreichen, sind die Geschwindigkeiten in dem Reaktor, durch die geeignete Auswahl des Einleitungsdruckes in Abhängigkeit von dem Reaktorradius und der Reaktionsgeschwindigkeit groß genug ausgewählt, um eine turbulente Strömung zu erzielen. Derzeitig angesetzt wird ein Reaktor mit einem etwa zehnfach höheren Strömungsfluss des eingesetzten Synthesegases als dem der Fermentationsbrühe. Zur Erhöhung der Durchflussgeschwindigkeiten wird in einigen Prozessläufen eine Rezirkulation des Offgases nach Abtrennung von Ethanol durch Trocknung durchgeführt, wobei hierdurch ein Effizienzverlust durch sinkende Reaktivgaskonzentrationen im Reaktor in Kauf genommen wird.

Bezugszeichen

Bezugzeichenliste für Figuren 1 und 2

| 1 | wässrige Lösung |
|---|---|
| 2 | Gas |
| 3 | Zelle |
| 4 | Mikrobiologie |
| 4a | Aktive Biomasse, unbeladen |
| 5 | Fermentationsbrühe |
| 5a | Aufzuchtfermenter |
| 6 | Garraum mit Eduktgas |
| 6a | Reaktor (Aerosolreaktor) |
| 7 | Niedergeschlagene/beladene Fermentationsbrühe |
| 8 | Biomasse aktiv (Rückführung, unbeladen) |
| 9 | Abscheider Biomasse |
| 10 | Inaktive Biomasse (Herausleitung aus Vorrichtung) |
| 11 | Produkt(e) (Herausleitung aus Vorrichtung) |
| 12 | Abscheider für Produkt(e) |
| 13 | Verdichter |
| 14 | Aerosolabscheider |
| 15 | Auffangfermenter |
| 16 | Rückführung aktive Eduktgase |
| 17 | Eduktgasquelle (Beispiel:Pyrolyseofen) |
| 18 | Rezirkulat Nährlösung-Aufzucht |
| 19 | Zellverdichter / Nährlösungstauscher |
| 20 | Zerstäuber |
| 21 | Biomasse, beladen |
| 22 | Ventil |

**Patentansprüche**

**1.** Verfahren zur mikrobiologischen Umsetzung von reaktiven Eduktgasen zu einem oder mehreren Produkten, **dadurch gekennzeichnet, dass** eine aktive Biomasse umfassende Zusammensetzung (4, 4a) als Aerosol in einen mit den reaktiven Eduktgasen gefüllten Raum (6) eingebracht wird.

**2.** Verfahren nach Anspruch 1, wobei die Zusammensetzung ferner ein Medium enthält.

**3.** Verfahren nach Anspruch 2, wobei das Medium ein wässriges Medium ist.

**4.** Verfahren nach einem der Ansprüche 1 bis 3, wobei die Zusammensetzung zusammen mit den Eduktgasen als Aerosol in den mit den reaktiven Eduktgasen gefüllten Raum (6) eingebracht wird.

**5.** Verfahren nach einem der Ansprüche 2 bis 4, wobei die aktive Biomasse von einer Fluidhülle aus einem Film des in der Zusammensetzung enthaltenden Mediums umgeben ist.

**6.** Verfahren nach Anspruch 5, wobei die Schichtdicke der Fluidhülle um die aktive Biomasse bei etwa 0,5 $\mu$m bis etwa 200 $\mu$m liegt, bevorzugt bei etwa 1 $\mu$m bis etwa 50 $\mu$m, weiter bevorzugt bei etwa 2 $\mu$m bis etwa 30 $\mu$m, besonders bevorzugt bei etwa 5 $\mu$m bis etwa 20 $\mu$m.

7. Verfahren nach einem der Ansprüche 1 bis 6, wobei die aktive Biomasse ein oder mehrere Mikroorganismen, Zellen und/oder Enzyme umfasst.

8. Verfahren nach Anspruch 7, wobei die Mikroorganismen ein oder mehrere sind ausgewählt aus der Gruppe umfassend: acetogene Bakterien, photosynthetisch tätige Mikroorganismen umfassend Algen und phototrophe Bakterien, methanotrophe Bakterien.

9. Verfahren nach einem der Ansprüche 1 bis 8, wobei die reaktiven Eduktgase eines oder mehrere sind aus der Gruppe umfassend Kohlenmonoxid, Kohlendioxid, Sauerstoff, Stickstoff, Wasserstoff, Ammoniak, Schwefelwasserstoff, Stickoxide, Methan, Synthesegas, Biogas, Formaldehyd, Kohlenwasserstoffe.

10. Verfahren nach einem der Ansprüche 1 bis 9, wobei das eine oder mehrere Produkte ausgewählt sind aus der Gruppe umfassend: Alkohole wie Ethanol, Methanolund Butanol, Kohlenwasserstoffe wie Methan, Wasserstoff, Carbonsäuren wie Essigsäure oder deren Ester, Carbonylverbindungen wie Aceton oder Formaldehyd, Stickstoff-Wasserstoff-Verbindungen wie Ammoniak, Biomasse oder relevante Bestandteile davon.

11. Vorrichtung zur mikrobiologischen Umsetzung reaktiver Eduktgase zu einem oder mehreren Produkten, umfassend

    (i) einen Reaktor (6a);
    (ii) eine Einrichtung zur Zuführung von aktiver Biomasse (4, 4a) in den Reaktor (6a);
    (iii) eine Einrichtung zur Zuführung von mindestens einem reaktiven Eduktgas (2) in den Reaktor; und
    (iv) eine Einrichtung zur Aerosolerzeugung (20);

derart eingerichtet, dass die aktive Biomasse durch die Einrichtung zur Aerosolerzeugung (20) als Aerosol in den Reaktor (6a) eingebracht wird und wobei das mindestens eine reaktive Eduktgas und die aktive Biomasse in die Gasphase (6) im Reaktor (6a) eingeführt werden.

12. Vorrichtung nach Anspruch 11, wobei die Einrichtung zur Zuführung von aktiver Biomasse (4, 4a) in den Reaktor (6a) und die Einrichtung zur Zuführung von mindestens einem reaktiven Eduktgas (2) in den Reaktor (6a) in der Einrichtung zur Aerosolerzeugung (20) münden und diese derart eingerichtet ist, die zugeleiteten Substanzen als Aerosol in die Gasphase (6) im Reaktor (6a) einzubringen.

13. Vorrichtung nach Anspruch 11 oder 12, wobei die Einrichtung zur Aerosolerzeugung (20) einen Ultraschallzerstäuber, einen Diffusor, oder eine Düse umfasst oder ist, wobei bei einer Düse die Einrichtung zur Aerosolerzeugung bevorzugt eine Piezodüse, eine Zweistoffdüse, eine Kegeldüse oder eine Zyklondüse umfasst oder ist.

14. Vorrichtung nach einem der Ansprüche 11 bis 13, wobei die Vorrichtung ferner umfasst eine oder mehrere Einrichtungen aus der Gruppe bestehend aus:

    (a) Lichtquellen umfassend Tageslichtfenster, Leuchtstofflampen, Metalldampflampen, Natriumdampf-Niederdrucklampen, Natriumdampf-Hochdrucklampen und Leuchtdioden;
    (b) Aufzuchtfermentern (5, 5a);
    (c) Verdichtern (13, 19);
    (d) Nährlösungstauschern (19);
    (e) Aerosolabscheidern (14);
    (f) Auffangfermentern (15);
    (g) Abscheidern (9, 12);
    (h) Pumpen;
    (i) Leitungen welche die Einrichtungen gemäß (ii) bis (iv) sowie (b) bis (h) miteinander und/oder mit dem Reaktor verbinden;
    (j) Leitungen zur Zurückleitung der reaktiven Eduktgase (16) in den Reaktor (6a);
    (k) Leitungen zur Zurückleitung des wässrigen Mediums (18) und/oder der aktiven Biomasse (8) in den Reaktor (6a) oder den Aufzuchtfermenter (5, 5a);
    (l) Leitungen zur Herausleitung der Produkte (11), aktiver Biomasse, inaktiver Biomasse (10), der Eduktgase und/oder des wässrigen Mediums aus der Vorrichtung;
    (m) Kondensationsvorrichtungen; und
    (n) Regeleinrichtungen umfassend Einrichtungen zur Erfassung und/oder Regelung von Temperatur, Druck, pH, Nährstoffen und Spurenelementen, Wassergehalt, Produktmenge, Durchflussmenge und/oder Aerosoltröpfchengröße;

15. Verwendung einer Vorrichtung nach einem der Ansprüche 11 bis 14 zur mikrobiologischen Umsetzung von reaktiven Eduktgasen zu einem oder mehreren Produkten.

**Claims**

1. A process for microbiological conversion of reactive educt gases to one or more products, **characterized in that** a composition comprising active biomass (4, 4a) is fed as aerosol in a space (6) filled with the reactive educt gases.

2. The method of claim 1, wherein the composition further comprises a medium.

3. The method of claim 2, wherein the medium is an aqueous medium.

4. The method according to any one of claims 1 to 3, wherein the composition is fed together with the educt gases as aerosol in the space (6) filled with the reactive educt gases.

5. The method according to any one of claims 2 to 4, wherein the active biomass is surrounded by a fluid shell of a film of the medium contained in the composition.

6. The method of claim 5, wherein the layer thickness of the fluid shell surrounding the active biomass is about 0.5 $\mu$m to about 200 $\mu$m, preferably about 1 $\mu$m to about 50 $\mu$m, more preferably about 2 $\mu$m to about 30 $\mu$m, most preferably about 5 $\mu$m to about 20 $\mu$m.

7. The method according to any one of claims 1 to 6, wherein the active biomass comprises one or more microorganisms, cells and/or enzymes.

8. The method according to claim 7, wherein the microorganisms are one or more selected from the group comprising: acetogenic bacteria, photosynthetic microorganisms comprising algae and phototrophic bacteria, methanotrophic bacteria.

9. The method according to any one of claims 1 to 8, wherein the reactive educt gases are one or more selected from the group comprising carbon monoxide, carbon dioxide, oxygen, nitrogen, hydrogen, ammonia, hydrogen sulfide, nitrogen oxides, methane, synthesis gas, biogas, formaldehyde, hydrocarbons.

10. The process according to any one of claims 1 to 9, wherein one or more product/s are selected from the group comprising: alcohols such as ethanol, methanol and butanol, hydrocarbons such as methane, hydrogen, carboxylic acids such as acetic acid or their esters, carbonyl compounds such as acetone or formaldehyde, nitrogen-hydrogen compounds such as ammonia, biomass or relevant components thereof.

11. A device for microbiological conversion of reactive educt gases to one or more product/s, comprising

(i) a reactor (6a);
(ii) a device for feeding active biomass (4, 4a) into the reactor (6a);
(iii) a device for feeding at least one reactive reactant gas (2) into the reactor; and
(iv) a device for generating an aerosol (20);
arranged such that the active biomass is fed as aerosol in the reactor (6a) by the device for generating an aerosol (20) and wherein the at least one reactive educt gas and the active biomass are fed in the gas phase (6) in the reactor (6a).

12. A device according to claim 11, wherein the device for feeding active biomass (4, 4a) into the reactor (6a) and the device for feeding at least one reactive educt gas (2) into the reactor (6a) emerge in the device for generating an aerosol (20) and this device is arranged such that the feeded substances are fed in the gas phase (6) of the reactor (6a) as aerosol.

13. A device according to claim 11 or 12, wherein the device for generating an aerosol (20) comprises or is an ultrasonic atomizer, a diffuser, or a nozzle, wherein in a nozzle, the device for aerosol generation preferably comprises or is a piezo nozzle, a two-substance nozzle, a cone nozzle or a cyclone nozzle.

14. The device according to any one of claims 11 to 13, wherein the device further comprises one or more device/s from the group consisting of:

(a) light sources comprising daylight windows, fluorescent lamps, metal vapor lamps, sodium vapor low pressure lamps, sodium vapor high pressure lamps and light emitting diodes;
(b) rearing fermenters (5, 5a);
(c) compressors (13, 19);
(d) nutrient solution exchangers (19);
(e) aerosol separators (14);
(f) collecting fermenters (15);
(g) separators (9, 12);
(h) pumps;
(i) lines connecting the devices according to (ii) to (iv) as well as (b) to (h) with each other and/or with the reactor;
(j) lines for recirculating the reactive reactant gases (16) to the reactor (6a);
(k) lines for recirculating the aqueous medium (18) and/or the active biomass (8) into the reactor (6a) or the rearing fermenter (5, 5a);
(l) lines for discharging the products (11), active biomass, inactive biomass (10), the educt gases and/or the aqueous medium from the device;
(m) condensation devices; and
(n) control devices comprising devices for detecting and/or controlling of temperature, pressure, pH, nutrients and trace elements, water content, product quantity, flow rate and/or aerosol droplet size.

15. Use of a device according to any one of claims 11 to 14 for the microbiological conversion of reactive educt gases to one or more products.

**Revendications**

1. Procédé de conversion microbiologique de gaz de départ réactifs en un ou plusieurs produits, **caractérisé en ce qu'**une composition comprenant de la biomasse active (4, 4a) est introduite sous forme d'aérosol dans un espace (6) rempli de gaz de départ réactifs.

2. Procédé selon la revendication 1, dans lequel la composition comprend en outre un milieu.

3. Procédé selon la revendication 2, dans lequel le milieu est un milieu aqueux.

4. Procédé selon l'une quelconque des revendications 1 à 3, dans lequel la composition est introduite avec les gaz de départ sous forme d'aérosol dans l'espace (6) rempli des gaz de départ réactifs.

5. Procédé selon l'une quelconque des revendications 2 à 4, dans lequel la biomasse active est entourée par une enveloppe fluide d'un film du milieu contenu dans la composition.

6. Procédé selon la revendication 5, dans lequel l'épaisseur de couche de l'enveloppe fluide entourant la biomasse active est d'environ 0,5 $\mu$m à environ 200 $\mu$m, de préférence d'environ 1 $\mu$m à environ 50 $\mu$m, plus préférablement d'environ 2 $\mu$m à environ 30 $\mu$m, le plus préférablement d'environ 5 $\mu$m à environ 20 $\mu$m.

7. Procédé selon l'une quelconque des revendications 1 à 6, dans lequel la biomasse active comprend un ou plusieurs micro-organismes, cellules et / ou enzymes.

8. Procédé selon la revendication 7, dans lequel les microorganismes sont un ou plusieurs choisis dans le groupe comprenant: bactéries acétogènes, microorganismes photosynthétiques comprenant des algues et des bactéries phototrophes, bactéries méthanotrophes.

9. Procédé selon l'une quelconque des revendications 1 à 8, dans lequel les gaz de départ réactifs sont un ou plusieurs choisis dans le groupe comprenant le monoxyde de carbone, le dioxyde de carbone, l'oxygène, l'azote, l'hydrogène, l'ammoniac, l'hydrogène sulfuré, les oxydes d'azote, le méthane, le gaz de synthèse, le biogaz, le formaldéhyde, les hydrocarbures.

10. Procédé selon l'une quelconque des revendications 1 à 9, dans lequel un ou plusieurs produits sont choisis dans le groupe comprenant: les alcools tels que l'éthanol, le méthanol et le butanol, les hydrocarbures tels que le méthane, l'hydrogène, les acides carboxyliques tels que l'acide acétique ou leurs esters, les composés carbonylés tels que l'acétone ou le formaldéhyde, les composés azote-hydrogène tels que l'ammoniac, la biomasse ou leurs composants pertinents.

11. Dispositif de conversion microbiologique de gaz de départ réactifs en un ou plusieurs produits, comprenant

    (i) un réacteur (6a);
    (ii) un dispositif d'alimentation de la biomasse active (4, 4a) dans le réacteur (6a);
    (iii) un dispositif d'alimentation d'au moins un gaz de départ réactif (2) dans le réacteur ; et
    (iv) un dispositif de génération d'aérosol (20);

agencé de telle sorte que la biomasse active est introduite sous forme d'aérosol dans le réacteur (6a) par le dispositif de génération d'aérosol (20) et dans laquelle l'au moins un gaz de départ réactif et la biomasse active sont introduits dans la phase gazeuse (6) dans le réacteur (6a).

12. Dispositif selon la revendication 11, dans lequel le dispositif d'alimentation de la biomasse active (4, 4a) dans le réacteur (6a) et le dispositif d'alimentation d'au moins un gaz de départ réactif (2) dans le réacteur (6a) émergent dans le dispositif pour générer un aérosol (20) et ce dispositif est agencé de telle sorte que les substances introduites sont introduites dans la phase gazeuse (6) du réacteur (6a) sous forme d'aérosol.

13. Dispositif selon la revendication 11 ou 12, dans lequel le dispositif de génération d'aérosol (20) comprend ou est un atomiseur à ultrasons, un diffuseur ou une buse, dans lequel dans une buse, le dispositif de génération d'aérosol comprend de préférence ou est une buse piezo, une buse à deux substances, une buse à cône ou une buse à cyclone.

14. Dispositif selon l'une quelconque des revendications 11 à 13, dans lequel le dispositif comprend en outre un ou plusieurs dispositif(s) du groupe comprenant:

    a) des sources lumineuses comprenant des fenêtres de jour, des lampes fluorescentes, des lampes à vapeur de métal, des lampes à vapeur de sodium à basse pression, des lampes à vapeur de sodium à haute pression et des diodes électroluminescentes;
    (b) des fermenteurs de l'élévage(5, 5a);
    (c) des compresseurs (13, 19);
    (d) des échangeurs de solution nutritive (19);
    (e) des séparateurs d'aérosol (14);
    (f) des fermenteurs de collection (15);
    (g) des séparateurs (9, 12);
    h) des pompes;
    (i) des conduite reliant les dispositifs selon (ii) à (iv) ainsi que (b) à (h) entre eux et / ou avec le réacteur;
    (j) des conduites pour faire recirculer les gaz de départ réactifs (16) dans le réacteur (6a);
    (k) des conduites pour recirculer le milieu aqueux (18) et / ou la biomasse active (8) dans le réacteur (6a) ou le fermenteur d'élevage (5, 5a);
    (l) des conduites d'évacuation des produits (11), de la biomasse active, de la biomasse inactive (10), des gaz de départ et / ou du milieu aqueux provenant du dispositif;
    (m) des dispositifs de condensation; et
    n) des dispositifs de commande comprenant des dispositifs de détection et / ou de contrôle de la température, de la pression, du pH, des nutriments et des oligo-éléments, de la teneur en eau, de la quantité de produit, du débit et / ou de la taille des gouttelettes aérosol.

15. Utilisation d'un dispositif selon l'une quelconque des revendications 11 à 14 pour la conversion microbiologique de gaz de départ réactifs en un ou plusieurs produits.

FIGUR 1

FIGUR 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20150031099 A1
- WO 2008154301 A1